(19) Europäisches Patentamt
European Patent Office
Office européen des brevets

(11) EP 1 852 431 A1

(12) **EUROPEAN PATENT APPLICATION**
published in accordance with Art. 158(3) EPC

(43) Date of publication:
**07.11.2007 Bulletin 2007/45**

(21) Application number: **06713167.2**

(22) Date of filing: **07.02.2006**

(51) Int Cl.:
*C07D 401/12* (2006.01) *A61K 31/4439* (2006.01)
*A61P 1/16* (2006.01) *A61P 1/18* (2006.01)
*A61P 9/10* (2006.01) *A61P 11/00* (2006.01)
*A61P 13/12* (2006.01) *A61P 17/06* (2006.01)
*A61P 19/02* (2006.01) *A61P 25/28* (2006.01)
*A61P 29/00* (2006.01) *A61P 31/04* (2006.01)
*A61P 37/06* (2006.01) *A61P 37/08* (2006.01)
*C07D 231/12* (2006.01) *C07D 401/14* (2006.01)

(86) International application number:
**PCT/JP2006/302024**

(87) International publication number:
**WO 2006/082975 (10.08.2006 Gazette 2006/32)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.02.2005 JP 2005029907**

(71) Applicant: **Mitsubishi Pharma Corporation**
**Chuo-ku,**
**Tokyo 103-8405 (JP)**

(72) Inventors:
- **NAKAMURA, Mitsuharu**
**MITSUBISHI PHARMA CORPORATION**
**Tokyo, 1038405 (JP)**
- **ISHIBUCHI, Seigo**
**MITSUBISHI PHARMA CORPORATION**
**Tokyo 1038405 (JP)**
- **OHTSUKA, Tatsuyuki**
**MITSUBISHI PHARMA CORPORATION**
**Tokyo 1038405 (JP)**
- **SUMICHIKA, Hiroshi**
**MITSUBISHI PHARMA CORPORATION**
**Tokyo, 1038405 (JP)**
- **SEKIGUCHI, Sumie**
**MITSUBISHI PHARMA CORPORATION**
**Tokyo 1038405 (JP)**
- **ISHIGE, Takayuki**
**MITSUBISHI PHARMA CORPORATION**
**Tokyo 1038405 (JP)**
- **UEDA, Naoko**
**MITSUBISHI PHARMA CORPORATION**
**Tokyo 1038405 (JP)**

(74) Representative: **Weber, Thomas**
**Von Kreisler Selting Werner**
**Bahnhofsvorplatz 1**
**50667 Köln (DE)**

# (54) OPTICALLY ACTIVE TETRAHYDRONAPHTHALENE DERIVATIVE

(57) A compound represented by the following formula (I), a pharmacologically acceptable salt thereof or a hydrate thereof or a solvate thereof, which shows not only a C5a receptor antagonistic activity but also high activity in the biological availability, as compared to its racemate.

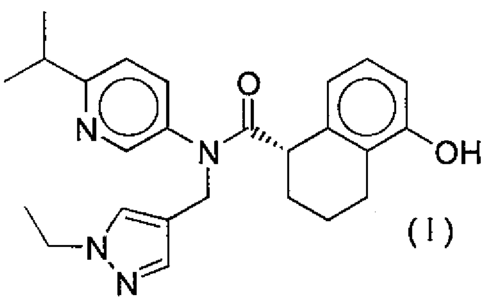

(I)

EP 1 852 431 A1

## Description

### Technical Field

The present invention relates to a novel optically active form of N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof (hereinafter to be abbreviated as the compound of the present invention), a pharmaceutical use thereof and a productive intermediate thereof.

### Background Art

When the complement system is activated, the protein of the complement system is subjected to enzymatic degradation and fragments having various physiological activities are produced. One of the fragments, complement component C5a, is a glycoprotein having a molecular weight of about 11,000, consists of 74 amino acids and has a strong inflammation inducing action. C5a shows a broad range of actions such as smooth muscle contraction, promotion of blood vessel permeability, migration of leukocyte, degranulation of leukocyte, production of reactive oxygen species, reinforcement of antibody production, induction of cytokine, TNF (tumor necrosis factor) and leukotriene production, and the like, and is said to be a causative substance of diseases such as autoimmune diseases (e.g., rheumatism and systemic lupus erythematosus and the like), sepsis, adult respiratory distress syndrome, chronic obstructive pulmonary disease, allergic diseases (e.g., asthma and the like), atherosclerosis, cardiac infarction, brain infarction, psoriasis, Alzheimer's disease, vital organ injury (e.g., pneumonia, nephritis, hepatitis, pancreatitis and the like) due to activation of leukocytes caused by ischemia reperfusion, trauma, burn, surgical invasion and the like, and the like [Annu. Rev. Immunol., vol. 12, pp. 775-808 (1994) (non-patent reference 1), Immunopharmacology, vol. 38, pp. 3-15 (1997) (non-patent reference 2), Curr. Pharm. Des., vol. 5, pp. 737-755 (1999) (non-patent reference 3) and IDrugs, vol. 2, pp. 686-693 (1999) (non-patent reference 4)].

Accordingly, a compound having a C5a receptor antagonistic action is expected as a novel non-steroid type antiinflammatory drug. In addition, it can be expected as a prophylactic or therapeutic drug of infectious diseases caused by bacteria or virus that invades via a C5a receptor.

Various patent applications regarding C5a antagonist have been published. Among them, WO02/22556 discloses racemate of N-[(1-ethyl-1H-pyrazol-4-yl)methyl]-5-hydroxy-N-(6-isopropylpyridin-3-yl)-1,2,3,4-tetrahydronaphthalene-1-carboxamide (patent reference 1, Example 89) but lacks a description relating to an optically active form.

[non-patent reference 1] Annu. Rev. Immunol., vol. 12, pages 775-808 (1994)
[non-patent reference 2] Immunopharmacology, vol. 38, pages 3-15 (1997)
[non-patent reference 3] Curr.Pharm.Des., vol. 5, pages 737-755 (1999)
[non-patent reference 4] IDrugs, vol. 2, pages 686-693 (1999)
[patent reference 1] WO02/22556

### Disclosure of the Invention

### Problems to be Solved by the Invention

The present invention aims at providing a novel compound superior in the C5a receptor antagonistic activity as compared to conventionally-known N-[(1-ethyl-1H-pyrazol-4-yl)methyl]-5-hydroxy-N-(6-isopropylpyridin-3-yl)-1,2,3,4-tetrahydronaphthalene-1-carboxamide (patent reference 1, Example 89), and further, in the biological availability, and a production intermediate therefor.

### Means of Solving the Problems

The present inventors have conducted intensive studies in view of the aforementioned problems and found that (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide is a compound superior in the C5a receptor antagonistic activity as compared to its racemate, and further, in the biological availability, which resulted in the completion of the present invention.

Accordingly, the present invention provides the following.

(1) (1S)-(-)-N-[(1-Ethyl-1H-pyrazol-4-yl)methyl]-5-hydroxy-N-(6-isopropylpyridin-3-yl)-1,2,3,4-tetrahydronaphthalene-1-carboxamide represented by the following formula (I)

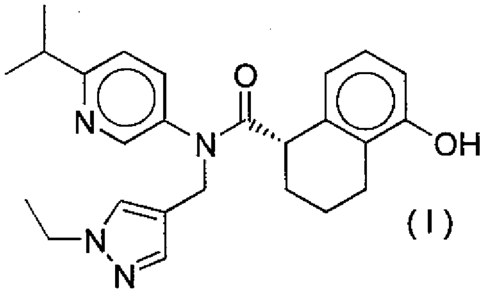

(I)

a pharmacologically acceptable salt thereof or a hydrate thereof or a solvate thereof.

(2) A crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.
(3) The crystal of (2), which is a crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.
(4) The crystal of (2) or (3), which shows a peak at a diffraction angle 2θ of about 14.6° (±0.2°) in powder X-ray diffraction spectrum.
(5) The crystal of any of (2) to (4), which shows a peak at a diffraction angle 2θ of about 10.0° (±0.2°) in powder X-ray diffraction spectrum.
(6) The crystal of any of (2) to (5), which shows a peak at a diffraction angle 2θ of about 8.5° (±0.2°) in powder X-ray diffraction spectrum.
(7) The crystal of any of (2) to (6), which shows peaks at a diffraction angle 2θ of about 20.1 and 23.2° (±0.2°, respectively) in powder X-ray diffraction spectrum.
(8) The crystal of any of (2) to (7), which shows characteristic peaks at a diffraction angle 2θ of about 8.5, 10.0, 14.6, 20.1 and 23.2° (each ±0.2°) in powder X-ray diffraction spectrum.
(9) The crystal of any of (2) to (8), which has a melting point (extrapolation-onset temperature) of about 171 to about 176°C.
(10) The crystal of any of (2) to (9), which has a melting point (extrapolation-onset temperature) of about 176°C.
(11) The crystal of any of (2) to (10), which has the physicochemical property shown in the following A and/or B:

A: having a powder X-ray diffraction pattern shown in Fig. 1
B: having a differential scanning calorimetry curve shown in Fig. 2.

(12) The crystal of (2) or (3), which shows a peak at a diffraction angle 2θ of about 5.9° (±0.2°) in powder X-ray diffraction spectrum.
(13) The crystal of (2), (3) or (12), which shows a peak at a diffraction angle 2θ of about 15.6° (±0.2°) in powder X-ray diffraction spectrum.
(14) The crystal of (2), (3), (12) or (13), which shows a peak at a diffraction angle 2θ of about 11.9° (±0.2°) in powder X-ray diffraction spectrum.
(15) The crystal of (2), (3), (12), (13) or (14), which shows a peak at a diffraction angle 2θ of about 21.3° (±0.2°) in powder X-ray diffraction spectrum.
(16) The crystal of (2), (3), (12), (13), (14) or (15), which shows characteristic peaks at a diffraction angle 2θ of about 5.9, 11.9, 15.6 and 21.3° (±0.2°, respectively) in powder X-ray diffraction spectrum.
(17) The crystal of (2), (3), (12), (13), (14), (15) or (16), which has a melting point (extrapolation-onset temperature) of about 96°C.
(18) The crystal of (2), (3), (12), (13), (14), (15), (16) or (17), which has the physicochemical property shown in the following C and/or D:

C: having a powder X-ray diffraction pattern shown in Fig. 3
D: having a differential scanning calorimetry curve shown in Fig. 4.

(19) An amorphous form of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.
(20) The amorphous form of (19), which is an amorphous form of (1S)-(-)-N-[(1-ethyl-1H-pyrazol-4-y1)methyl]-5-hydroxy-N(6-isopropylpyridin-3-yl)-1,2,3,4-tetrahydronaphthalene-1-carboxamide.
(21) The amorphous form of (19) or (20), which has the physicochemical property shown in the following E:

E: having a powder X-ray diffraction pattern shown in Fig. 5.

(22) The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of (1), whose pharmacologically acceptable salt is a hydrochloride.
(23) The -(1S)-(-)-N-[(1-ethyl-1H-pyrazol-4-yl)methyl]-5-hydroxy N-(6-isopropylpyridin-3-yl)-1,2,3,4-tetrahydronaphthalene-1-carboxamide, a pharmacologically acceptable salt thereof or a hydrate thereof or a solvate thereof of (1) or (22), whose pharmacologically acceptable salt is a monohydrochloride.
(24) The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of (1), whose pharmacologically acceptable salt is a hydrobromide.
(25) The (1S)-(-)-N-[(1-ethyl-1H-pyrazol-4-yl)methyl]-5-hydroxy-N-(6-isopropylpyrid in-3-yl)-1,2,3,4-tetrahydronaphthalene-l-carboxamide, a pharmacologically acceptable salt thereof or a hydrate thereof or a solvate thereof of (1) or (24), whose pharmacologically acceptable salt is a monohydrobromide.
(26) A pharmaceutical agent comprising the compound of any of (1) to (25).
(27) A pharmaceutical composition comprising the compound of any of (1) to (25), and a pharmaceutically acceptable additive.
(28) A drug for the prophylaxis or treatment of a disease caused by binding of C5a with a C5a receptor, which comprises the compound of any of (1) to (25) as an active ingredient.
(29) The drug of (28), wherein the disease caused by the binding of C5a with a C5a receptor is autoimmune disease, sepsis, adult respiratory distress syndrome, chronic obstructive pulmonary disease, allergic disease, atherosclerosis, myocardial infarction, cerebral infarction, psoriasis, Alzheimer's disease, or organ injury caused by leukocyte activation due to ischemia reperfusion, trauma, burn or surgical invasion.
(30) An anti-inflammatory agent comprising the compound of any of (1) to (25) as an active ingredient.
(31) A C5a receptor antagonist comprising the compound of any of (1) to (25) as an active ingredient.
(32) The antagonist of (31), which is a drug for the prophylaxis and/or treatment of infection with bacterium or virus that invades via a C5a receptor.
(33) A drug for the prophylaxis and/or treatment of rheumatoid arthritis, which comprises, as an active ingredient, (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.
(34) The drug of (33), wherein the active ingredient is (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.
(35) The drug of (33) or (34), wherein the active ingredient is a crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.
(36) The drug of any of (33) to (35), wherein the active ingredient shows characteristic peaks at a diffraction angle $2\theta$ of about 8.5, 10.0, 14.6, 20.1 and 23.2° (each $\pm 0.2°$) in powder X-ray diffraction spectrum.
(37) The drug of any of (33) to (36), wherein the active ingredient has a melting point (extrapolation-onset temperature) of about 176°C.
(38) 1-Ethyl-N-(6-isopropylpyridin-3-yl)-1H-pyrazole-4-carboxamide or N-(6-isopropylpyridin-3-yl)-1-vinyl-1H-pyrazole-4-carboxamide, which is represented by the following formula (II)

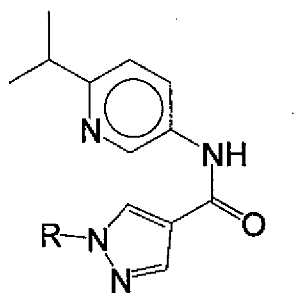

(II)

wherein R is ethyl or vinyl.
(39) [(1-Ethyl-1H-pyrazol4-yl)methyl](6-isopropylpyridin-3-yl)amine or (6-isopropylpyridin-3-yl)[(1-vinyl-1H-pyrazol-4-yl)methyl]amine, which is represented by the following formula (III)

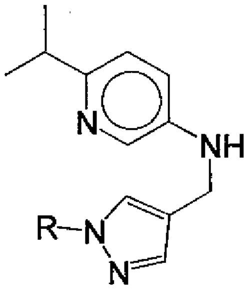

(III)

wherein R is ethyl or vinyl.

(40) A 1-ethyl-1H-pyrazole compound represented by the following formula (IV)

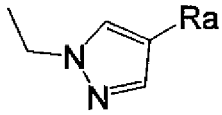

(IV)

wherein Ra is hydroxymethyl or formyl.

(41) Ethyl 1-(2-chloroethyl)-1H-pyrazole-4-carboxylate represented by the following formula (V)

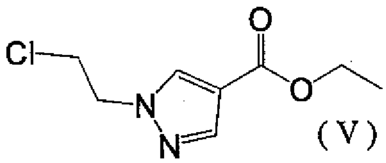

(V)

(42) A 1-vinyl-1H-pyrazole compound represented by the following formula (VI)

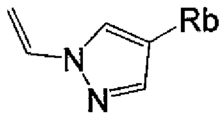

(VI)

wherein Rb is ethoxycarbonyl, hydroxycarbonyl, hydroxymethyl or formyl.

(43) (1S)-(-)-N-[(1-Ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of (1), wherein the solvate is a methanol solvate.

(44) (1S)-(-)-N-[(1-Ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of (1) or (43), wherein the solvate is a monomethanol solvate.

## Effect of the Invention

(1S)-(-)-N-[(-Ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof, which is the compound of the present invention, is a compound showing higher activity as compared to its racemate not only in the C5a receptor antagonistic activity but also in the biological availability.

**Brief Description of the Drawings**

Fig. 1 shows an XRD pattern of a free base · Form-I crystal.
Fig. 2 shows a DSC curve of a free base·Form-I crystal.
Fig. 3 shows an XRD pattern of the compound obtained in Example 22.
Fig. 4 shows a DSC curve of the compound obtained in Example 22.
Fig. 5 shows an XRD pattern of the compound obtained in Example 23.
Fig. 6 shows a DSC curve of the compound obtained in Example 23.
Fig. 7 shows a DSC curve of the compound obtained in Example 24.
Fig. 8 shows a DSC curve of the compound obtained in Example 25.
Fig. 9 shows an XRD pattern of the compound obtained in Example 26.
Fig. 10 shows an XRD pattern of the compound obtained in Example 26 after drying under reduced pressure at 40°C for 35 min.
Fig. 11 shows the results of a dissolution test.

**Best Mode for Embodying the Invention**

The subject matter of the present invention rests in an (S)-isomer of N-[(1-ethyl-1H-pyrazol-4-y1)methyl]-5-hydroxy-N-(6-isopropylpyridin-3-yl)-1,2,3,4-tetrahydronaphthalene-1-carboxamide, which is represented by the above-mentioned formula (I), a pharmacologically acceptable salt thereof or a hydrate thereof or a solvate thereof, and a pharmaceutical agent comprising the same as an active ingredient, and a production intermediate for (S)-isomer of N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.

While the compound of the present invention represented by the above-mentioned formula (I) can be produced, for example, by following method, the methods shown in the below-mentioned Examples, and the like, these production methods are examples, and the production method is not limited to them. The production methods exemplified below may be used alone or in combination and a conventional method may be further combined. Where necessary, each compound is protected or deprotected by a conventional method. The resultant product obtained in each of the following steps can be isolated and produced by a conventional method.

Method 1: Production method 1 of compound (III')

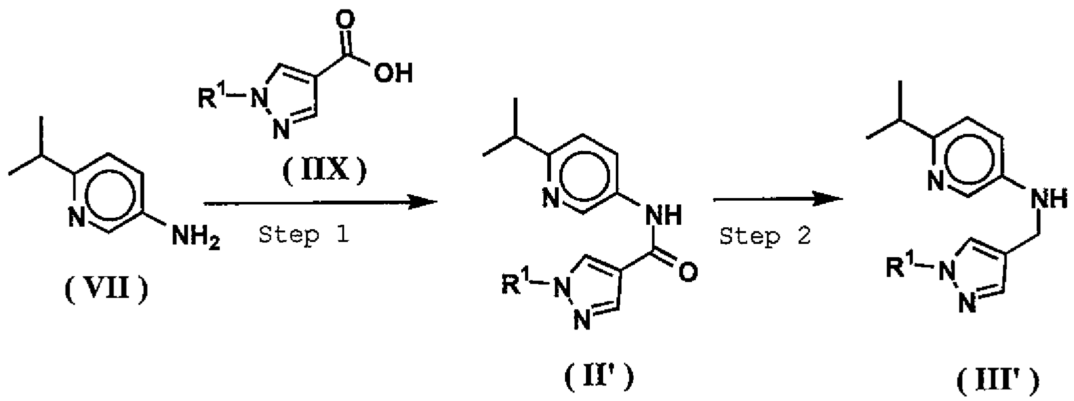


(IIX) (VII) (II') (III')

wherein $R^1$ is ethyl group optionally having substituent(s), vinyl group optionally having substituent(s), ethynyl group optionally having substituent(s) or nitrogen-protecting group.

Compound (III') can be produced by reacting compound (VII) or a salt thereof with compound (IIX) or a reactive derivative thereof without solvent or in an appropriate solvent to give compound (II'), and reacting compound (II') with a reducing agent in an appropriate solvent (step 1, 2).

Step 1 can be performed according to a known amidation method or peptide synthetic method, and the like, for example, in the presence of a condensation agent (carbodiimides (N,N-dicyclohexylcarbodiimide, 1-ethyl-3-(3-dimethylaminopropyl)carbodiimide etc.), diphenylphosphoryl azide, carbonyldiimidazole, 1-benzotriazolyloxytris(dimethylamino)phosphonium hexafluorophosphate (Bop reagent), 2-chloro-N-methylpyridinium iodide-tributylamine system (Mukaiyama method), N-cyclohexylcarbodiimide-N'-methylpolystyrene etc.), in an inert solvent or without solvent, preferably at -78°C to 80°C. Step 1 can be also performed in the presence of a base {for example, organic bases (triethylamine, N-methylmorpholine, pyridine, dimethylaniline, 4-dimethylaminopyridine etc.), inorganic bases (sodium hydrogencarbonate, potassium carbonate, sodium hydroxide, potassium hydroxide, sodium hydride), n-butyllithium, sodium tert-butoxide, potassium tert-butoxide etc.)}, and the like. Generally, the reaction of step 1 is completed within 24 hr.

Compound (III') can be also produced by converting compound (IIX) to another reactive derivative. When the reactive derivative of compound (IIX) is an acid halide (e.g., acid chloride, acid bromide and the like) or an acid anhydride (e.g., a symmetric acid anhydride, a mixed acid anhydride with lower alkyl carbonate, alkylphosphoric acid and the like), the reaction with compound (VII) can be generally carried out in an inert solvent or without solvent, at -78°C to the refluxing temperature of the solvent.

When, as a reactive derivative of compound (IIX), what is called an active ester (4-nitrophenyl ester, 4-chlorobenzyl ester, 4-chlorophenyl ester, pentafluorophenyl ester, succinic acid imide ester, benzotriazole ester, 4-dimethylsulfoniumphenyl ester and the like) is used, the reaction can be generally carried out in an inert solvent or without solvent, at -78°C to the refluxing temperature of the solvent.

The inert solvent used for the above-mentioned amidation reaction includes hydrocarbons such as hexane, benzene, toluene and the like; halogenated hydrocarbons such as chloroform, dichloromethane, dichloroethane and the like; ethers such as tetrahydrofuran, dioxane and the like; acetates; ketones such as acetone, methyl ethyl ketone and the like; alcohols such as methanol, ethanol, isopropyl alcohol and the like; amides such as N,N-dimethylformamide, dimethylacetamide and the like; acetonitrile, dimethyl sulfoxide, water, a mixed solvent thereof and the like.

Examples of the reducing agent used for the reduction reaction in step 2 include lithium aluminum hydride, borane, sodium bis(2-methoxyethoxy)aluminum hydride, sodium borohydride and the like. In addition, as an additive, iodine, a Lewis acid (e.g., aluminum chloride, boron trifluoride diethyl ether complex and the like), sulfuric acid and the like can be used. Examples of the solvent used for the reduction reaction include tetrahydrofuran, diethyl ether, hexane, toluene and the like, and the solvent may be a mixed solvent of these. The reaction temperature varies depending on the solvent, and it is generally -78°C to the refluxing temperature of the solvent. The reaction time varies depending on the reaction temperature, and it is generally 1 hr to 24 hr.

Method 2: Production method 2 of compound (III')

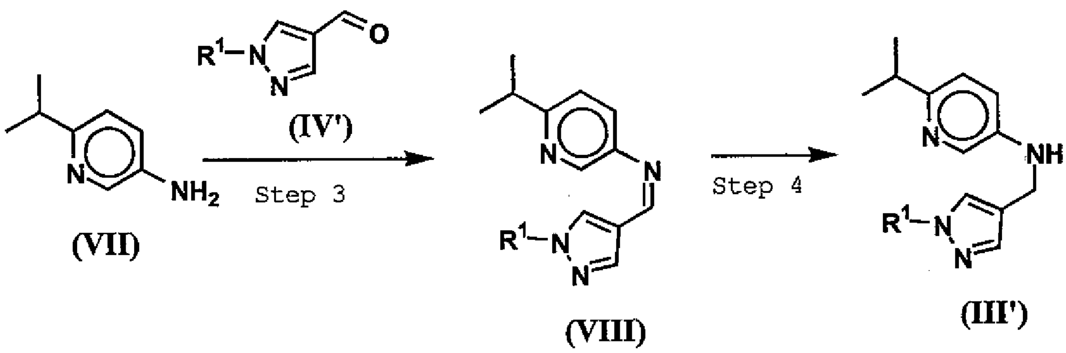


wherein $R^1$ is as defined above.

Compound (III') can be produced by subjecting compound (VII) and compound (IV') to dehydration condensation without solvent or in an appropriate solvent to give compound (VIII), and reacting compound (VIII) with a reducing agent in an appropriate solvent (steps 3, 4).

The dehydration condensation of compound (VII) and compound (IV') in step 3 can be carried out in the presence of a dehydrating agent or by removing the produced water from the reaction system using a Dean-Stark apparatus.

As the dehydrating agent used for this reaction, a conventional dehydrating agent can be used. Examples of the dehydrating agent include anhydrous magnesium sulfate, molecular sieves and the like. Examples of the solvent used for the reaction include methylene chloride, chloroform, benzene, toluene, xylene and the like. The reaction temperature varies depending on the solvent, and it is generally 0°C to the refluxing temperature of the solvent. The reaction time varies depending on the reaction temperature, and it is generally 1 hr to 24 hr.

The compound to be used in step 3 which is a compound (IV') wherein $R^1$ is vinyl optionally having substituent (s) can be synthesized, for example, according to the methods of Examples 6 to 10, via compounds (V) and (VI).

Examples of the reducing agent used in step 4 include sodium borohydride, sodium triacetoxyborohydride, sodium cyanoborohydride, formic acid, sodium formate and the like. In addition, when sodium triacetoxyborohydride or

sodium cyanoborohydride is used as a reducing agent, removal of water using the dehydrating agent or Dean-Stark trap in step 3 can be omitted. Examples of the solvent used for the reaction include water, methanol, ethanol, propanol, tetrahydrofuran, dioxane, methylene chloride, chloroform, dichloroethane, acetic acid, benzene, toluene, xylene and the like, and the solvent may be a mixed solvent of these. The reaction temperature varies depending on the solvent, and it is generally 0°C to 150°C. The reaction time varies depending on the reaction temperature, and it is generally 1 hr to 24 hr.

Method 3: Production method of compound (I)

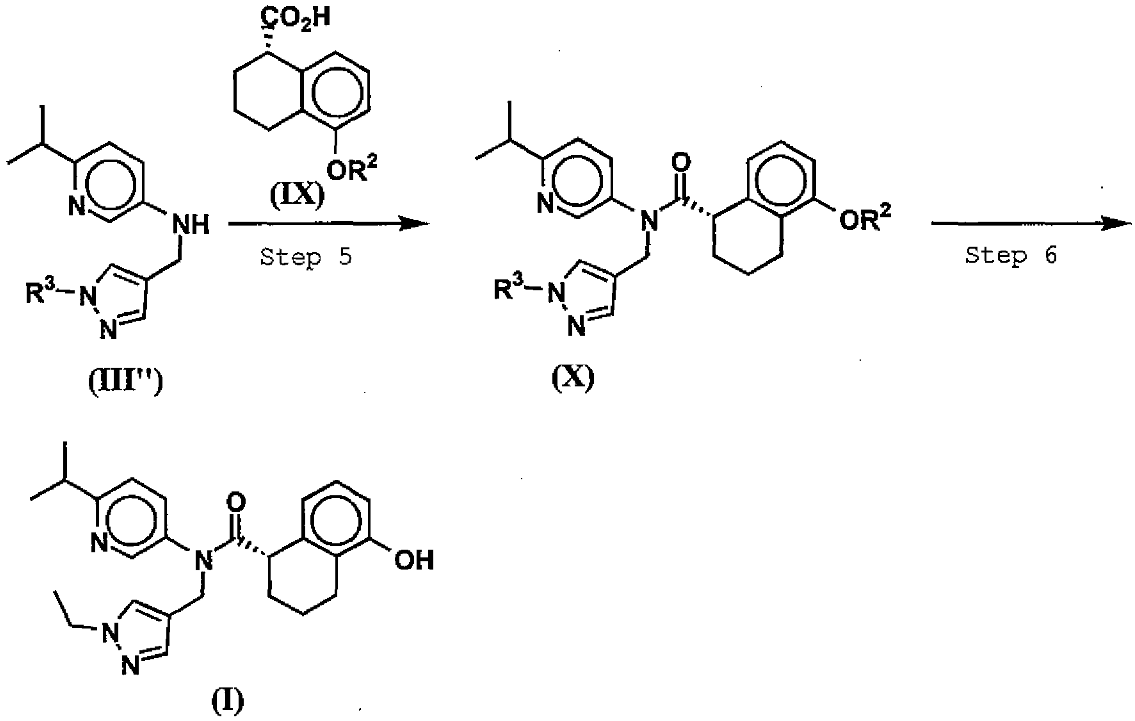


(IX) (III'') (X) (I)

wherein $R^2$ is hydroxyl-protecting group, and $R^3$ is ethyl group optionally having substituent(s), vinyl group optionally having substituent(s) or ethynyl group optionally having substituent(s).

Compound (I) can be produced by reacting compound (III'') obtained according to the above-mentioned Method 1 or 2 with compound (IX) under appropriate conditions to give compound (X), eliminating the protecting group of compound (X), and reducing the resulting compound as necessary (steps 5, 6).

The reaction of compound (IX) or a reactive derivative thereof with compound (III'') or a salt thereof in step 5 can be carried out in the same manner as in step 1.

The elimination of the protecting group $R^2$ in step 6 can be carried out according to conventional methods such as hydrolysis, acid treatment, catalytic hydrogenation using a metallic catalyst (palladium carbon, Raney nickel and the like), and the like, depending on the kind of the protecting group.

The return of $R^3$ to the ethyl group can be carried out according to conventional methods such as hydrolysis, acid treatment, tetrabutylammonium fluoride (TBAF), lithium aluminum hydride, lithium/liquid ammonia, catalytic hydrogenation using a metallic catalyst (palladium carbon, Raney nickel and the like), and the like, depending on the kind of $R^3$. This reaction may be carried out simultaneously with the deprotection of the hydroxyl group (elimination of $R^2$), or may be carried out before or after the deprotection reaction.

Method 4: Production method of compound (X)

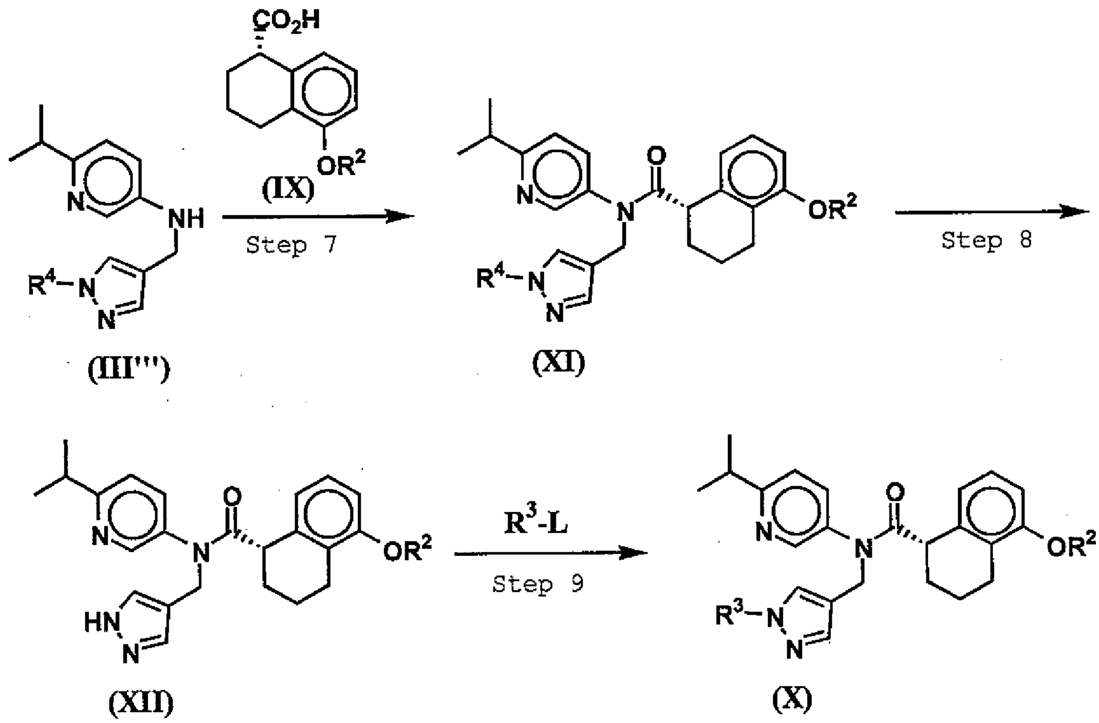

(III''') (XI) (XII) (X)

wherein $R^2$ and $R^3$ are as defined above, $R^4$ is a nitrogen-protecting group, and L is a leaving group such as halogen atom, methanesulfonyloxy or p-toluenesulfonyloxy and the like.

Compound (X) can also be produced from compound (III''') obtained according to the above-mentioned Method 1 or 2, via steps 7 to 9. The obtained compound (X) can be converted to compound (I) according to the above-mentioned step 6 in Method 3.

The reaction of compound (IX) or a reactive derivative thereof with compound (III''') or a salt thereof in step 7 can be carried out in the same manner as in step 1.

The elimination of the protecting group $R^4$ in step 8 can be carried out according to a conventional method such as hydrolysis, acid treatment, catalytic hydrogenation using a metallic catalyst (palladium carbon, Raney nickel and the like), and the like, depending on the kind of the protecting group.

The reaction in step 9 can be carried out in a solvent that does not inhibit the reaction, or without solvent and, where necessary, in the presence of a base {for example, organic bases (triethylamine, N-methylmorpholine, pyridine, dimethylaniline, 4-dimethylaminopyridine etc.), inorganic bases (sodium hydride, sodium hydrogencarbonate, potassium carbonate, potassium phosphate, sodium hydroxide), n-butyllithium, sodium tert-butoxide, potassium tert-butoxide and the like), metallic catalysts (palladium, copper, copper iodide, copper cyanide and the like) and the like. Examples of the solvent used in step 9 include hydrocarbons such as hexane, benzene, toluene and the like; halogenated hydrocarbons such as chloroform, dichloromethane, dichloroethane and the like; ethers such as diethyl ether, THF, dioxane and the like; esters such as acetate ester and the like; ketones such as acetone, methyl ethyl ketone and the like; alcohols such as methanol, ethanol, isopropyl alcohol and the like; amides such as DMF, DMA and the like; acetonitrile, DMSO and a mixed solvent thereof and the like.

Method 5: Production method 1 of compound (XV)

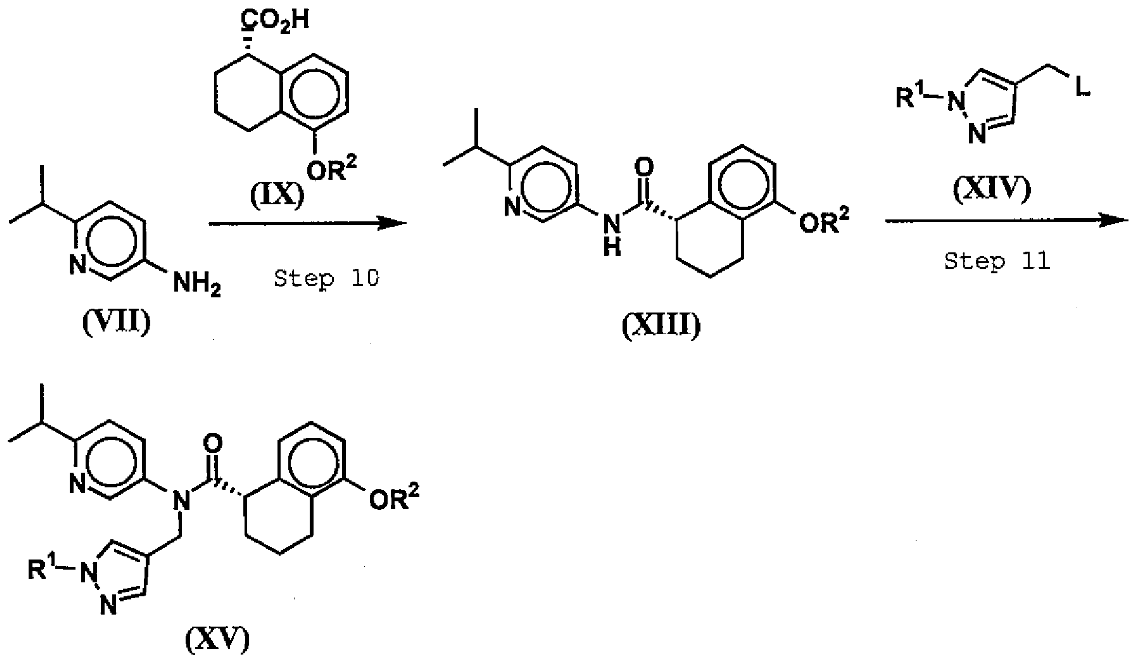


(VII) (IX) (XIII) (XIV) (XV)

wherein $R^1$, $R^2$ and L are as defined above.
Compound (XV) can be produced by reacting compound (VII) with compound (IX) to give compound (XIII), and reacting compound (XIII) with compound (XIV) under appropriate conditions (steps 10, 11).

The reaction of compound (VII) or a salt thereof with compound (IX) or a reactive derivative thereof in step 10 can be carried out in the same manner as in step 1.

Step 11 is performed in a solvent that does not inhibit the reaction, in the presence of a base {for example, organic bases (triethylamine, N-methylmorpholine, pyridine, dimethylaniline, 4-dimethylaminopyridine etc.), inorganic bases (sodium hydride, sodium carbonate, potassium carbonate, sodium hydroxide), n-butyllithium, sodium tert-butoxide, potassium tert-butoxide, lithium diisopropylamide and the like} and the like at -78°C to the refluxing temperature of the solvent. Examples of the solvent used in step 11 include hydrocarbons such as hexane, benzene, toluene and the like; halogenated hydrocarbons such as chloroform, dichloromethane, dichloroethane and the like; ethers such as diethyl ether, THF, dioxane and the like; esters such as acetate ester and the like; ketones such as acetone, methyl ethyl ketone and the like; alcohols such as methanol, ethanol, isopropyl alcohol and the like; amides such as DMF, DMA and the like; acetonitrile, DMSO and a mixed solvent thereof and the like.

Method 6: Production method 2 of compound (XV)

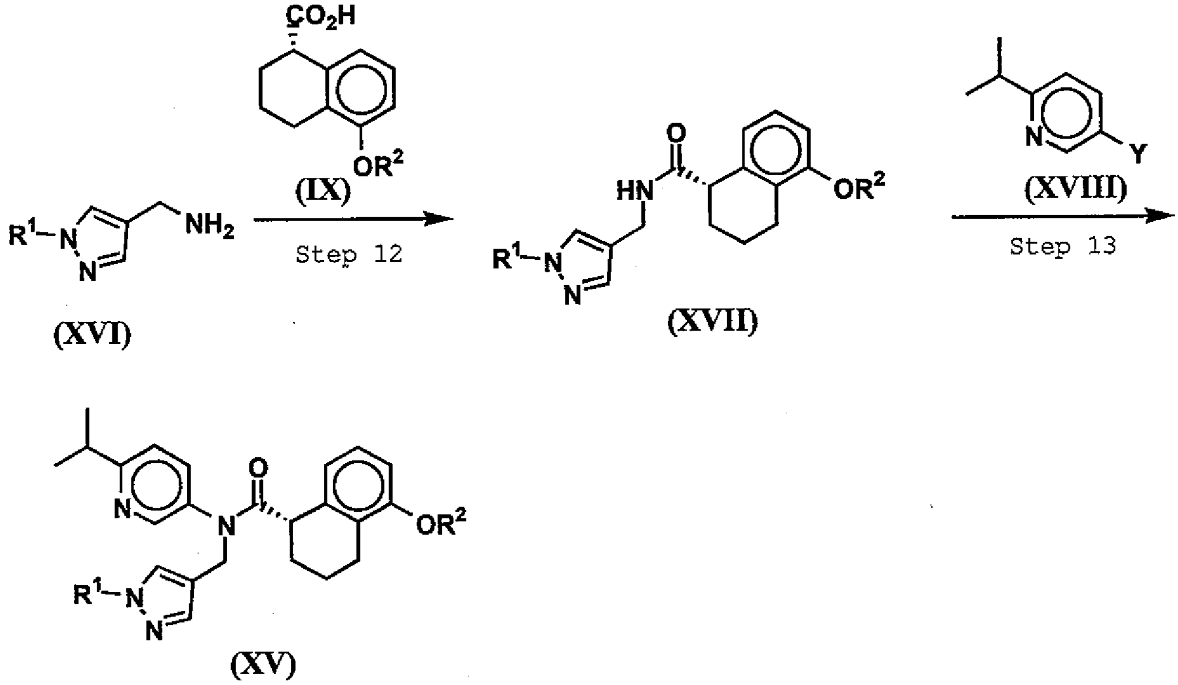


(XVI) (IX) (XVII) (XVIII) (XV)

wherein $R^1$ and $R^2$ are as defined above, and Y is halogen atom, trifluoromethanesulfonyloxy and the like. Compound (XV) can be produced by reacting compound (XVI) with compound (IX) to give compound (XVII), and reacting compound (XVII) with compound (XVIII) under appropriate conditions (steps 10, 11).

The reaction of compound (XVI) or a salt thereof with compound (IX) or a reactive derivative thereof in step 12 can be carried out in the same manner as in step 1.

Step 13 is performed in a solvent that does not inhibit the reaction, in the presence of a base, for example, an organic base (triethylamine, N-methylmorpholine, pyridine, dimethylaniline, 4-dimethylaminopyridine and the like), an inorganic base (sodium hydride, sodium hydrogencarbonate, potassium carbonate, potassium phosphate, cesium carbonate, sodium hydroxide and the like) and, where necessary, in the presence of a catalyst (copper, copper cyanide, copper iodide and the like) or 1,2-diamine ligand (ethylenediamine, N,N'-dimethylethylenediamine, 1,2-diaminocyclohexene and the like) at -20°C to the refluxing temperature of the solvent. Examples of the solvent used in step 13 include hydrocarbons such as hexane, benzene, toluene and the like; halogenated hydrocarbons such as chloroform, dichloromethane, dichloroethane and the like; ethers such as diethyl ether, THF, dioxane and the like; esters such as acetate and the like; ketones such as acetone, methyl ethyl ketone and the like; alcohols such as methanol, ethanol, isopropyl alcohol and the like; amides such as DMF, DMA and the like; nitrobenzene, acetonitrile, DMSO, water, a mixed solvent thereof and the like. Step 13 can be also performed according to the method described in a literature (J. AM. CHEM. SOC. 2002, 124, 7421-7428).

Compound (XV) obtained according to Method 5 or 6 can be converted to compound (I) according to Method 3 or 4.

The compounds described earlier, which are represented by the formulas (II), (III), (IV), (V) and (VI) are novel compounds and can be produced, for example, by the methods described in the below-mentioned Examples. These compounds can be led to compound (I) by the methods described in the below-mentioned Examples and Preparation Examples. Thus, they are useful as production intermediates for compound (I). In addition, a methanol solvate of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide is a novel compound, which can be (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide by drying, thereby being useful as a production intermediate for compound (I).

In the present invention, examples of the substituent of the "optionally having substituent(s)" include halogen atom, trialkylsilyl group, hydroxyl group optionally having protecting group and the like. Examples of the nitrogen-protecting group include benzyl, substituted benzyl, benzyloxycarbonyl, tert-butyloxycarbonyl and the like. Examples of the hydroxyl-protecting group include methyl, benzyl, substituted benzyl, benzyloxycarbonyl and the like. Examples of the halogen atom include chlorine atom, bromine atom, iodine atom, fluorine atom and the like.

In the present invention, the pharmacologically acceptable salt includes salts with inorganic acids such as hydrochloric acid, hydrobromic acid, sulfuric acid, phosphoric acid, nitric acid and the like, salts with organic acids such

as acetic acid, propionic acid, succinic acid, glycolic acid, lactic acid, malic acid, tartaric acid, citric acid, maleic acid, fumaric acid, methanesulfonic acid, benzenesulfonic acid, p-toluenesulfonic acid, camphorsulfonic acid, ascorbic acid and the like, salts with alkali metal (lithium, sodium, potassium etc.), salts with alkaline earth metal (calcium, magnesium etc.), salts with metals such as aluminum and the like, salts with organic bases such as piperidine, piperazine, morpholine, diethanolamine, ethylenediamine and the like. They can be obtained by treating with the aforementioned acid, alkali metal, alkaline earth metal, metal or organic base in, where necessary, an appropriate solvent (methanol, ethanol etc.). When the obtained compound is not a hydrate or solvate, it can be converted to a hydrate or solvate by a treatment with water, a water-containing solvent or other solvent.

A preferable embodiment of the compound of the present invention is, for example, a crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof, and a more preferable embodiment is, for example, a crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide. The crystal preferably has the powder X-ray diffraction pattern shown in Fig. 1 and/or the differential scanning calorimetry (DSC) curve shown in Fig. 2. Here, the characteristic peaks of the powder X-ray diffraction pattern in terms of diffraction angle 2θ include 8.5, 10.0, 14.6, 20.1 and/or 23.2° (each $\pm$0.2°). In addition, the melting point (extrapolation-onset temperature) in DSC is, for example, about 171°C - about 176°C, preferably about 176°C. Other preferable crystal may be one having the powder X-ray diffraction pattern shown in Fig. 3 and/or the DSC curve shown in Fig. 4. Here, the characteristic peaks of the powder X-ray diffraction pattern in terms of diffraction angle 2θ include 5.9, 11.9, 15.6 and/or 21.3°(each $\pm$0.2°). In addition, the melting point (extrapolation-onset temperature) in DSC is, for example, about 96°C.

Other preferable embodiment of the compound of the present invention is, for example, an amorphous form of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof, more preferably an amorphous form of (1S)-(-)-N-[(1ethyl-1H-pyrazol-4-yl)methyl]-5-hydroxy-N-(6-isopropylpyridin-3-yl)-1,2,3,4-tetrahydronaphthalene-1-carboxamide.

Still another preferable embodiment of the compound of the present invention is, for example, hydrochloride or hydrobromide of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, more preferably monohydrochloride or monohydrobromide. Here, the extrapolation-onset temperature of the melting and/or decomposition peak by DSC is about 172°C for monohydrochloride and about 189°C for monohydrobromide.

In the present invention, the compound of the present invention includes a prodrug converted to the aforementioned compound of the formula (I) by metabolism in the living body, and an active metabolite of the compound of the formula (I).

Some of the terms used in the present specification are defined below.

The "prophylactic drug" means a drug to be administered to a healthy subject before onset of a disease, which is, for example, a drug administered for the purpose of preventing the onset of the disease.

The "therapeutic drug" means a drug to be administered to a subject (patient) diagnosed by a physician to have developed a disease, which is, for example, a drug administered for the purpose of alleviation of disease or symptom, or recovery of health. When the drug is to be administered to a patient, even if the administration purpose is prevention of aggravation of a disease or symptom, or prevention of a fit, the drug is a therapeutic drug.

The "substances that bind to a C5a receptor" means C5a, a decomposition product of C5a (e.g., C5a desArg wherein the carboxy terminal arginine of C5a has been deleted), and known or unknown substances, which are other than C5a, having affinity for C5a receptor.

The "C5a receptor antagonist" is a substance that inhibits binding of a C5a receptor with a "substance that binds with a C5a receptor".

The "C5a receptor antagonistic action" means an action that inhibits a reaction that causes some physiological changes (e.g., increase of intracellular $Ca^{2+}$, and the like) by binding of a "substance that binds with a C5a receptor" via the C5a receptor with a cell that expresses the C5a receptor.

The compound of the present invention shows a C5a receptor antagonistic action, and is useful as a drug for the prophylaxis or treatment of diseases caused by binding of C5a with a C5a receptor, for example, autoimmune diseases such as rheumatoid arthritis, systemic lupus erythematosus and the like; sepsis; adult respiratory distress syndrome; chronic obstructive pulmonary disease; allergic diseases such as asthma and the like; atherosclerosis; myocardial infarction; cerebral infarction; psoriasis; Alzheimer's disease; vital organ injury caused by leukocyte activation induced by ischemia reperfusion, trauma, burn, surgical invasion and the like (e.g., pneumonia, nephritis, hepatitis, pancreatitis and the like) and the like. Here, examples of effective autoimmune diseases include rheumatoid arthritis. Since C5a has a strong inflammation-inducing action, the compound of the present invention is useful as an anti-inflammatory agent, and further, a drug for the prophylaxis and/or treatment of an infectious disease caused by invasion of bacterium or virus via a C5a receptor.

When the compound of the present invention is used as the aforementioned prophylactic and/or therapeutic drug, it is generally administered systemically or topically and orally or parenterally. The dose to patients varies depending on the age, body weight, sex, general health conditions, treatment effect, diet, administration time, administration method, clearance rate, combination of drugs, the condition of the disease under treatment and the like. It is generally desirably in the range of from 0.1 mg to 500 mg per dose for an adult by oral administration once to several times a day, or in the range of from 0.01 mg to 200 mg per dose for an adult by parenteral administration (preferably intravenous administration) once to several times a day. The dose is more desirably increased or decreased as appropriate according to the condition of patients.

The compound of the present invention can be used orally or parenterally, for example, by inhalation, rectal administration, topical administration and the like as a pharmaceutical composition or preparation (e.g., powder, granule, tablet, pill, capsule, syrup, elixir, suspension, solution and the like), wherein at least one compound of the present invention can be used alone or used upon admixing with a pharmaceutically acceptable carrier (excipient, binder, disintegrant, corrigent, corrective, emulsifier, diluent and/or dissolution aids and the like).

A pharmaceutical composition can be prepared according to a general method. In the present specification, by the parenteral is meant subcutaneous injection, intravenous injection, intramuscular injection, intraperitoneal injection, drip and the like. A composition for injection, such as sterile suspension for injection and oil suspension can be prepared using a suitable dispersing agent, wetting agent, or suspending agent according to a method known in the art.

A solid composition for oral administration is exemplified by tablet, pill, capsule, powder, granule and the like. In the above-mentioned solid composition, one or more active compounds can be admixed with at least one additive.

In addition, the above-mentioned composition can contain further additives such as lubricant, preservative, antioxidant, disintegrant, stabilizer, dissolution aids, binder, thickener, sweetener, flavor, perfume and the like.

Where necessary, the tablet and pill may be coated with a film of a gastric soluble or enteric material, or may be coated with two or more layers.

The liquid composition for oral administration comprises pharmaceutically acceptable solution, emulsion, syrup, elixir and the like, and may contain a generally used inactive diluent. This composition may contain, besides the inactive diluent, auxiliaries such as wetting agent, suspending agent, and the like, sweetening agent, flavor, perfume and preservative. Other compositions for oral administration are, for example, spray agent containing one or more active substances and formulated by a method known per se.

The composition for injection for parenteral administration may comprise sterile aqueous or non-aqueous solution, suspension and emulsion. The above-mentioned composition may further contain auxiliaries such as preservative, wetting agent, emulsifier, dispersing agent, stabilizer and dissolution aids. These can be sterilized by, for example, filtration through a bacteria-retaining filter, addition of microbicide or irradiation.

The composition for injection can be also used by producing a sterile solid composition and dissolving, for example, the lyophilized product in sterile water or sterile solvent for injection before use.

Other composition for parenteral administration include external solution, ointment, liniment, suppository and the like, containing one or more active substances and formulated by a conventional method.

The suppository for rectal administration can be produced by admixing the drug and a suitable non-irritant vehicle, such as a substance which is solid at ambient temperature but shows liquid properties at the temperature of intestinal tract and which melts in the rectum to release the drug.

## Examples

The present invention is specifically explained in the following by referring to Examples and the like, which are not to be construed as limitative.

The chemical shift of $^1$H-NMR was expressed as relative delta (δ) value in parts per million (ppm) using tetramethylsilane (TMS) as the internal standard. For the coupling constant, obvious multiplicity is shown using s (singlet), d (doublet), t (triplet), q (quartet), sept (septet), m (multiplet), dd (double doublet), brs (broad singlet) and the like in hertz (Hz).

Thin-layer chromatography was performed using silica gel manufactured by Merck, and column chromatography was performed using silica gel manufactured by Fuji Silysia Chemical.

## Preparation Example 1

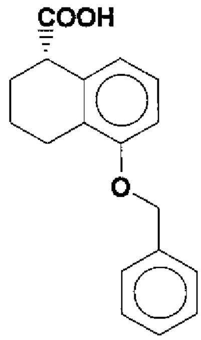

5-Benzyloxy-1,2,3,4-tetrahydronaphthalene-1-carboxylic acid (178 g) and (3S)-(-)-3-aminopyrrolidine (54.3 g) were dissolved in methanol (700 mL), and the solvent was evaporated under reduced pressure to give crude crystals (229.6 g). The crystals were recrystallized from a mixed solvent of THF and water to give white crystals (86 g). The crystals (48 g) were added to 1 mol/L-hydrochloride, and the mixture was extracted with ethyl acetate. The organic layer was dried over anhydrous magnesium sulfate. The solvent was evaporated under reduced pressure to give (1S)-(-)-5-benzyloxy-1,2,3,4-tetrahydronaphthalene-1-carboxylic acid (36.8 g). $[\alpha]_D$ -51.8° (24°C, c=1. 0, methanol)

**Example 1**

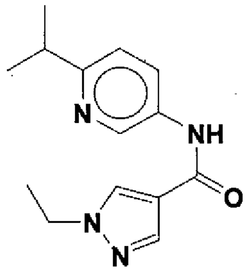

To a solution of 1-ethyl-1H-pyrazole-4-carboxylic acid (9.80 g) in 1,2-dichloroethane (50 mL) was added thionyl chloride (7.66 mL), and the mixture was stirred at 60°C for 3 hr. The reaction mixture was concentrated under reduced pressure, toluene was added to the residue, and the mixture was concentrated again under reduced pressure. A solution of the residue in 1,2-dichloroethane (25 mL) was added to a solution of 3-amino-6-isopropylpyridine (9.52 g) in 1,2-dichloroethane (100 mL) under ice-cooling. The temperature was raised to room temperature, and the mixture was stirred at the same temperature for 2 hr. The reaction mixture was poured into saturated aqueous sodium hydrogencarbonate, and the mixture was partitioned and extracted with chloroform. The organic layer was washed with saturated brine, and dried over anhydrous magnesium sulfate. The solvent was evaporated, and the residual solid was suspended in isopropyl ether and collected by filtration to give 1-ethyl-N-(6-isopropylpyridin-3-yl)-1H-pyrazole-4-carboxamide (11.4 g) as brown powder crystals.
$^1$H-NMR ($CDCl_3$)δ: 1.27(6H,d,J=6.9Hz), 1.46(3H,t,J=7.2Hz), 3.02(1H,sept,J=6.9Hz), 4.14(2H,q,J=7.2Hz), 7.13(1H,d, J=8.6Hz), 7.93(1H,s), 7.98(1H,s), 8.12(1H,dd,J=2.6,8.6Hz), 8.53-8.57(2H,m)

**Example 2**

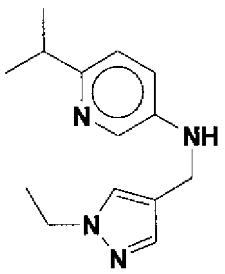

1-Ethyl-N-(6-isopropylpyridin-3-yl)-1H-pyrazole-4-carboxamide (11.4 g) was dissolved in 100 mL of borane·THF complex/1 mol/L-THF solution ($BH_3$·THF complex/1M THF solution), and the mixture was heated under reflux for 4 hr. After cooling, the reaction mixture was added to 1 mol/L-hydrochloric acid, and the mixture was heated under reflux for several minutes. After cooling, the reaction mixture was poured into saturated aqueous sodium hydrogencarbonate, and the mixture was partitioned and extracted with ethyl acetate. The organic layer was washed with saturated brine, and dried over anhydrous magnesium sulfate. The solvent was evaporated, and the residue was purified by silica gel column chromatography to give [(1-ethyl-1H-pyrazol-4-yl)methyl](6-isopropylpyridin-3-yl)amine (9.86 g) as a pale-yellow oil.
$^1$H-NMR($CDCl_3$)δ: 1.27(6H,d,J=6.9Hz), 1.46(3H,t,J=7.2Hz), 2.96(1H,sept,J=6.9Hz), 3.77-3.90(1H,brs), 4.08-4.20(4H, m), 6.89(1H,dd,J=2.6,8.6Hz), 6.9(1H,d,J=8.6Hz), 7.37(1H,s), 7.47(1H,s), 8.01(1H,d,J=2.6Hz)

**Example 3**

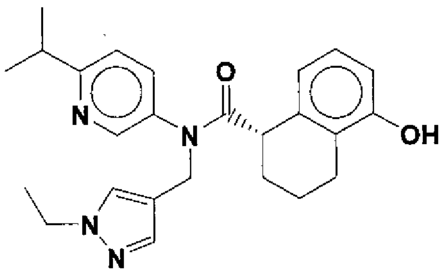

To a solution of (1S)-(-)-5-benzylaxy-1,2,3,4-tetrahydronaphthalene-1-carboxylic acid (1.98 g) in methylene chloride (15 mL) were added thionyl chloride (0.77 mL) and a catalytic amount of dimethylformamide, and the mixture was heated under reflux with stirring for 1.5 hr. The reaction mixture was concentrated under reduced pressure, toluene was added to the residue, and the mixture was concentrated again under reduced pressure. A solution of the residue in 1,2-dichloroethane (15 mL) was added to a solution of [(1-ethyl-1H-pyrazol-4-yl)methyl](6-isopropylpyridin-3-yl)amine (1.71 g), pyridine (1.13 mL), and 4-dimethylaminopyridine (DMAP) (8.6 mg) in 1,2-dichloroethane (15 mL) under ice-cooling, the temperature was raised to room temperature, and the mixture was stirred at the same temperature for 16 hr. The reaction mixture was poured into 4% aqueous sodium hydrogencarbonate solution, and the mixture was partitioned and extracted with chloroform. The organic layer was washed with saturated brine, and dried over anhydrous magnesium sulfate. The solvent was evaporated, and the residue was purified by silica gel column chromatography to give (1S)-5-benzyloxy-N-[(1-ethyl-1H-pyrazol-4-yl)methyl]-N-(6-isopropylpyridin-3-yl)-1,2,3,4-tetrahydronaphthalene-1-carboxamide (2.86 g) as a white amorphous material. $^1$H-NMR($CDCl_3$)δ: 1.31(6H,d,J=6.9Hz), 1.45(3H,t,J=7.3Hz), 1.40-1.57(1H,m), 1.75-2.07(3H,m), 2.65-2.77(2H,m), 2.72(1H,sept,J=6.9Hz), 3.64(1H,t,J=6.2Hz), 4.14(2H,q,J=7.3Hz), 4.61(1H,d,J=13.9Hz), 4.85(1H,d,J=13.9Hz), 5.03(2H,s), 6.53(1H,d,J=7.7Hz), 6.72(1.H,d,J=7.7Hz), 7.03(1H,t,J=7.7Hz), 7.24-7.43(9H,m), 8.39(1H,d,J=1.5Hz)
To a solution of the amide form (2.86 g) obtained by the above-mentioned operation in methanol (30 mL) were added 10% palladium carbon (300 mg) and ammonium formate (1.78 g) under a nitrogen atmosphere, and the mixture was stirred at room temperature for 3 days. The reaction mixture was filtered and the solvent was evaporated. Water was added to the residue and the mixture was partitioned and extracted with ethyl acetate. The organic layer was washed with saturated brine, and dried over anhydrous magnesium sulfate. The solvent was evaporated, and the residue was

recrystallized from a mixed solvent of ethyl acetate and hexane to give (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide (1.27 g) as a white powder crystal (hereinafter (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide having the same physicochemical properties as the crystal is sometimes to be referred to as free base•Form-I crystal).

optical purity 99.9%e.e.

analysis conditions

column :CHIRALCEL OD(0.46 $\phi\times$25 cm, DAICEL CHEMICAL INDUSTRIES, LTD.)
developing solvent: hexane/isopropanol=85/15
flow rate: 1.0 mL/min
column oven temperature: 40°C
UV detection: 254 nm
retention time: 18.5 min.

specific rotation of free base•Form-I crystal $[\alpha]_D$ -93.0° (21°C, c=1.0, methanol).

powder X-ray diffraction (XRD) analysis of free base•Form-I crystal
XRD pattern was measured under the following conditions. apparatus: RINT2200/Ultima+ (Rigaku Corporation)
conditions:

X-ray vacuum tube: Cu $K_\alpha 1$
electric current: 40 mA
electric voltage: 40 kV
scanning speed: 1 - 4°/min
scanning range: 2θ=2 - 40°

Powder X-ray diffraction pattern is shown in Fig. 1.
The characteristic peaks of the crystal in terms of diffraction angle 2θ were 8.5, 10.0, 14.6, 20.1 and 23.2° (each ±0.2°).

Differential scanning calorimetry (DSC) of free base•Form-I crystal
The obtained compound (3 mg) was set on differential scanning calorimeter DSC821e (METTLER-TOLEDO K.K.), and the measurement was performed at a scanning rise rate of 10°C/min (25 - 200°C, nitrogen 40 mL/min). As a result, the melting point (extrapolation-onset temperature) was observed at 176°C. The DSC curve is shown in Fig. 2

**Example 4**

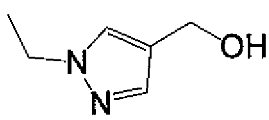

To a solution of 1-ethyl-1H-pyrazole-4-carboxylic acid (9.44 g) in tetrahydrofuran (10 mL) was added 100 mL of a borane· tetrahydrofuran complex/1 mol/L-tetrahydrofuran solution ($BH_3$·THF complex/1M THF solution), and the mixture was stirred overnight at room temperature. 1 mol/L hydrochloric acid (150 mL) was added, and the mixture was heated under reflux for 30 min. After cooling, the reaction mixture was poured into saturated aqueous sodium hydrogencarbonate, and the mixture was partitioned and extracted with ethyl acetate. The organic layer was washed with saturated brine, and dried over anhydrous magnesium sulfate. The solvent was evaporated to give (1-ethyl-1H-pyrazol-4-yl)methanol (2.84 g) as a pale-yellow oil.
$^1$H-NMR($CDCl_3$)δ: 1.48(3H,t,J=7.5Hz), 4.16(2H,q,J=7.5Hz), 4.59(2H,s), 7.42(1H,s), 7.49(1H,s).

**Example 5**

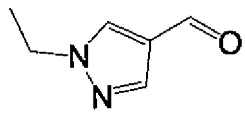

To (1-ethyl-1H-pyrazol-4-yl)methanol (2.84 g) were added methylene chloride (124 mL), manganese dioxide (13.5 g) and anhydrous magnesium sulfate (3.7 g), and the mixture was stirred at room temperature. After confirmation of completion of the reaction, the reaction solution was filtrated. The solvent was evaporated to give (1-ethyl-1H-pyrazol-4-yl)carbaldehyde (2.61 g) as a pale-yellow oil.
$^1$H-NMR($CDCl_3$)δ: 1.54(3H,t,J=7.4Hz), 4.23(2H,q,J=7.4Hz), 7.95(1H,s), 7.97(1H,s), 9.85(1H,s).

**Example 6**

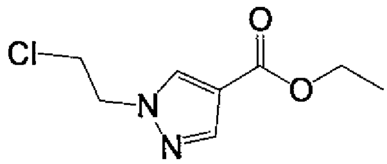

To a solution of ethyl 1H-pyrazole-4-carboxylate (0.46 g) in dichloroethane (50 mL) were added tetra-n-butylammonium hydrogensulfate (0.24 g) and 50% (W/W) aqueous sodium hydroxide solution (15 mL), and the mixture was stirred at room temperature. After confirmation of completion of the reaction, the reaction mixture was partitioned between water and chloroform. The organic layer was washed with saturated brine, and dried over anhydrous magnesium sulfate. The solvent was evaporated to give ethyl 1-(2-chloroethyl)-1H-pyrazole-4-carboxylate (0.72 g).
$^1$H-NMR($CDCl_3$)δ:1.35(3H,t,J=7.2Hz),3.91(2H,t,J=5.7Hz), 4.30(2H,q,J=7.2Hz),4.44(2H,t,J=5.7Hz),7.96(1H,s),7.99 (1H,s) MS (ESI)m/z:203 $[MH]^+$.

**Example 7**

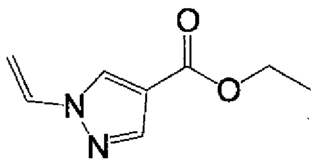

To ethyl 1-(2-chloroethyl)-1H-pyrazole-4-carboxylate (0.72 g) were added dimethyl sulfoxide (40 mL) and 1,8-diazabicyclo[5.4.0]undec-7-ene (2 g), and the mixture was stirred under heating at 80°C. After confirmation of completion of the reaction, the reaction mixture was poured into water, and the mixture was partitioned and extracted with ethyl acetate. The organic layer was washed with saturated brine, and dried over anhydrous magnesium sulfate. The solvent was evaporated, and the residue was purified by silica gel column chromatography to give ethyl 1-vinyl-1H-pyrazole-4-carboxylate (0.344 g).
$^1$H-NMR($CDCl_3$)δ: 1.36 (3H, t, J=7.2Hz), 4.31 (2H, q, J=7.2Hz), 5.00 (1H, dd, J=1.2, 9.0Hz), 5.66 (1H, dd, J=1.2, 15.9Hz), 7.03 (1H, dd, J=9.0, 15.9Hz), 7.99 (1H, s), 8.08 (1H, s).

**Example 8**

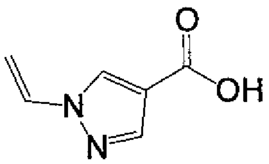

To ethyl 1-vinyl-1H-pyrazole-4-carboxylate (0.344 g) were added ethanol (5 mL) and 1 mol/L aqueous sodium hydroxide solution (2.5 mL), and the mixture was stirred under heating at 50°C. After confirmation of completion of the reaction, the reaction mixture was acidified with 1 mol/L hydrochloric acid, and the mixture was partitioned and extracted with ethyl acetate. The organic layer was washed with saturated brine, and dried over anhydrous magnesium sulfate. The solvent was evaporated to give 1-vinyl-1H-pyrazole-4-carboxylic acid (0.253 g).
$^1$H-NMR($CDCl_3$)δ: 5.00(1H,d,J=8.7Hz), 5.74(1H,d,J=15.6Hz), 7.27(1H,dd,J=8.7,15.6Hz), 7.96(1H,s), 8.57(1H,s), 12.59 (1H,brs).

**Example 9**

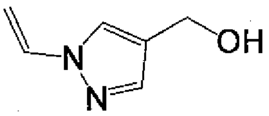

Ethyl 1-vinyl-1H-pyrazole-4-carboxylate (2.0 g) was dissolved in diethyl ether (51 mL), and 1 mol/L diisobutylaluminum hydride/toluene solution (DIBAL) (26 mL) was added dropwise in a nitrogen stream under stirring at -78°C. The reaction temperature was raised to room temperature, and the mixture was stirred at the same temperature for 3 hr. Under ice-cooling, methanol (1.9 mL), diethyl ether (3.2 mL) and saturated aqueous solution (12.8 mL) of potassium sodium tartrate tetrahydrate (Rochelle salt) were successively added. After stirring at room temperature for 1 hr, the reaction mixture was filtrated, and the filtrate was dried over anhydrous magnesium sulfate. The solvent was evaporated, and the residue was purified by silica gel column chromatography to give (1-vinyl-1H-pyrazol-4-yl)methanol (1.04 g).
$^1$H-NMR($CDCl_3$) δ: 1.64(1H,t,J=5.4Hz), 4.62(2H,d,J=5.4Hz), 4.84(1H,d,J=9.0z), 5.48(1H,d,J=16.2Hz), 7.02(1H,dd, J=9.0,16.2Hz), 7.61(1H,s), 7.63(1H,s).

**Example 10**

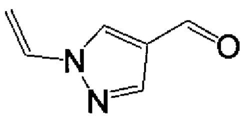

To (1-vinyl-1H-pyrazol-4-yl)methanol (1.04 g) were added methylene chloride (47 mL), manganese dioxide (5.03 g) and anhydrous magnesium sulfate (1.39 g), and the mixture was stirred at room temperature. After confirmation of completion of the reaction, the reaction solution was filtrated, and the solvent was evaporated to give 1-vinyl-1H-pyrazole-4-carbaldehyde (0.83 g).
$^1$H-NMR($CDCl_3$)δ: 5.08(1H,dd,J=1.5,8.7Hz), 5.75(1H,dd,J=1.5, 15.6Hz), 7.06(1H,dd,J=8.7,15.6Hz), 8.07(1H,s), 8.10 (1H,s), 9.91(1H,s).

**Preparation Example 2**

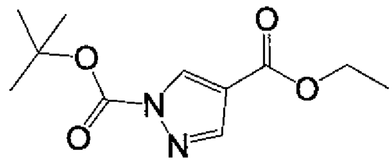

Ethyl 1H-pyrazole-4-carboxylate (14.3 g) and 4-dimethylaminopyridine (40 mg) were dissolved in tetrahydrofuran (80 mL), a solution of di-tert-butyl dicarbonate (23.0 g) in tetrahydrofuran (20 mL) was added at room temperature, and the mixture was stirred at the same temperature for 7 hr. The reaction mixture was evaporated under reduced pressure and the residue was purified by silica gel column chromatography to give 1-tert-butyl 4-ethyl 1H-pyrazole-1,4-dicarboxylate (28.9 g).
$^1$H-NMR($CDCl_3$)δ: 1.37(3H,t,J=7.2Hz), 1.67(9H,s), 4.33(2H,q,J=7.2Hz), 8.06(1H,s), 8.55(1H,s).

**Preparation Example 3**

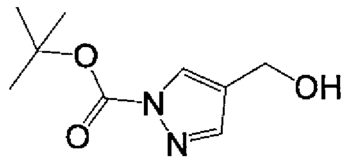

1-tert-Butyl 4-ethyl 1H-pyrazole-1,4-dicarboxylate (11.3 g) was dissolved in diethyl ether (200 mL), and 1 mol/L diisobutylaluminum hydride/toluene solution (DIBAL) (100 mL) was added dropwise in a nitrogen stream under stirring at -78°C. The reaction temperature was raised to room temperature, and the mixture was stirred at the same temperature for 2 hr. Under ice-cooling, methanol (7.5 mL), diethyl ether (12.5 mL) and a saturated aqueous solution (50 mL) of potassium sodium tartrate tetrahydrate (Rochelle salt) were successively added. After stirring at room temperature for 1 hr, the reaction mixture was filtrated and the filtrate was dried over anhydrous magnesium sulfate. The solvent was evaporated, and the residue was purified by silica gel column chromatography to give tert-butyl 4-(hydroxymethyl)-1H-pyrazole-1-carboxylate (3.55 g).
$^1$H-NMR($CDCl_3$)δ: 1.65(9H,s), 1.89(1H,brs), 4.63(2H,s), 7.72(1H,s), 8.06(1H,s).

**Preparation Example 4**

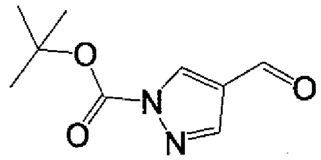

To tert-butyl 4-(hydroxymethyl)-1H-pyrazole-1-carboxylate (3.55 g) were added methylene chloride (100 mL), manganese dioxide (10.76 g) and anhydrous magnesium sulfate (2.97 g), and the mixture was stirred at room temperature. After confirmation of completion of the reaction, the reaction solution was filtrated, and the solvent was evaporated to give tert-butyl 4-formyl-1H-pyrazole-1-carboxylate (3.6 g).
$^1$H-NMR($CDCl_3$)δ: 1.68(9H,s), 8.13(1H,s), 8.62(1H,s), 9.97(1H,s).

**Example 11**

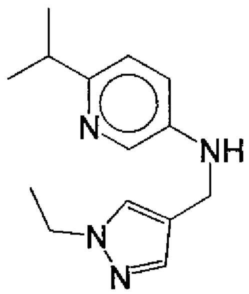

(1-Ethyl-1H-pyrazol-4-yl)carbaldehyde (0.091 g) and (6-isopropylpyridin-3-yl)amine (0.1 g) were dissolved in 1,2-dichloroethane (4 mL), acetic acid (0.04 mL) and sodium triacetoxyborohydride (0.311 g) were added, and the mixture was stirred overnight at room temperature. The reaction mixture was poured into saturated aqueous sodium hydrogencarbonate, and the mixture was partitioned and extracted with ethyl acetate. The organic layer was washed with saturated brine, and dried over anhydrous magnesium sulfate. The solvent was evaporated, and the residue was purified by silica gel column chromatography to give [(1-ethyl-1H-pyrazol-4-yl)methyl](6-isopropylpyridin-3-yl)amine (0.174 g) as a pale-yellow oil.
MS (ESI)m/z:245$[MH]^+$.

**Example 12**

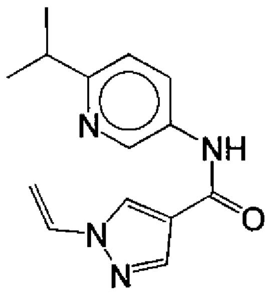

1-Vinyl-1H-pyrazole-4-carboxylic acid (0.767 g) and (6-isopropylpyridin-3-yl)amine (0.756 g) were dissolved in dimethylformamide (30 mL), N-hydroxybenzotriazole (0.748 g) and 1-ethyl-3-(3'-dimethylaminopropyl)carbodiimide hydrochloride (1.07 g) were added, and the mixture was stirred overnight at room temperature. The reaction mixture was poured into water, and the mixture was partitioned and extracted with ethyl acetate. The organic layer was washed with saturated brine, and dried over anhydrous magnesium sulfate. The solvent was evaporated, and the residue was purified by silica gel column chromatography to give N-(6-isopropylpyridin-3-yl)-1-vinyl-1H-pyrazole-4-carboxamide (1.28 g).
$^1$H-NMR($CDCl_3$)δ: 1.29(6H,d,J=6.9Hz), 3.06(1H,sept,J=6.9Hz), 5.02(1H,dd,J=1.3,8.8Hz), 5.67(1H,dd,J=1.3,15.7Hz), 7.04(1H,dd,J=8.8,15.7Hz), 7.20(1H,d,J=8.6Hz), 7.86(1H,brs), 8.00 (1H, s), 8.15(1H,d,J=2.5Hz), 8.18(1H,s), 8.54(1H,d, J=2.3Hz),
MS(ESI)m/z:257$[MH]^+$.

**Example 13**

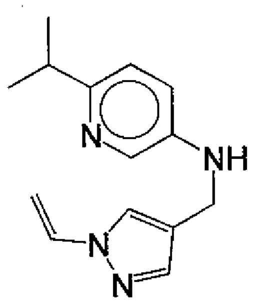

N-(6-Isopropylpyridin-3-yl)-1-vinyl-1H-pyrazole-4-carboxamide (1.54 g) was suspended in toluene (15 mL), sodium bis(2-methoxyethoxy)aluminum hydride-toluene solution (65+wt.%)(Red-Al) (6.0 mL) was gradually added under heating at 65°C and stirring, and the mixture was stirred at the same temperature with heating for 40 min. The reaction mixture was ice-cooled, ethyl acetate (100 mL) was added, and the mixture was stirred for 10 min. Under ice-cooling, to the reaction mixture was added 4N-aqueous sodium hydroxide solution, and the mixture was partitioned and extracted with ethyl acetate. The organic layer was washed twice with saturated brine, and dried over anhydrous sodium sulfate. The solvent was evaporated, and the residue was purified by silica gel column chromatography to give (6-isopropylpyridin-3-y1)[(1-vinyl-1H-pyrazol-4-yl)methyl]amine (1.11 g) as white crystals.
$^1$H-NMR($CDCl_3$)δ: 1.25(6H,d,J=6.9Hz), 2.96(1H,sept,J=6.9Hz), 3.85(1H,brs), 4.22(2H,d,J=3.6Hz), 4.82(1H,d,J=9.3Hz), 5.46(1H,d,J=15.9Hz), 6.89(1H,dd,J=3.0,8.4Hz), 6.96-7.04(2H,m), 7.58(2H,s), 8.02(1H,d,J=2.7Hz)
MS(ESI)m/z:243[MH]$^+$.

**Example 14**

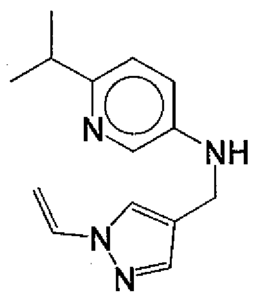

1-Vinyl-1H-pyrazole-4-carbaldehyde (0.3 g) and (6-isopropylpyridin-3-yl)amine (0.335 g) were dissolved in 1,2-dichloroethane (12 mL), acetic acid (0.14 mL) and sodium triacetoxyborohydride (1.04 g) were added, and the mixture was stirred overnight at room temperature. The reaction mixture was poured into saturated aqueous sodium hydrogencarbonate, and the mixture was partitioned and extracted with ethyl acetate. The organic layer was washed with saturated brine, and dried over anhydrous magnesium sulfate. The solvent was evaporated, and the residue was purified by silica gel column chromatography to give (6-isopropylpyridin-3-yl)[(1-vinyl-1H-pyrazol-4-yl)methyl]amine (0.574 g).
MS(ESI)m/z:243[MH]$^+$.

**Example 15**

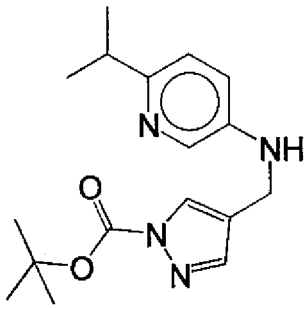

tert-butyl 4-formyl-1H-pyrazole-1-carboxylate (1.0 g) and (6-isopropylpyridin-3-yl)amine (0.694 g) were dissolved in 1,2-dichloroethane (25 mL), acetic acid (0.29 mL) and sodium triacetoxyborohydride (2.16 g) were added, and the mixture was stirred overnight at room temperature. The reaction mixture was poured into saturated aqueous sodium hydrogencarbonate, and the mixture was partitioned and extracted with ethyl acetate. The organic layer was washed with saturated brine, and dried over anhydrous magnesium sulfate. The solvent was evaporated, and the residue was purified by silica gel column chromatography to give tert-butyl 4-{[(6-isopropylpyridin-3-yl)amino]methyl}-1H-pyrazole-1-carboxylate (1.46 g).
$^1$H-NMR($CDCl_3$)δ: 1.26(6H,d,J=6.9Hz), 1.65(9H,s), 2.96(1H,sept,J=6.9Hz), 3.80-3.90(1H,m), 4.23(2H,d,J=6.0Hz), 6.88 (1H,dd,J=3.0,8.4Hz), 6.99(1H,d,J=8.4Hz), 7.70(1H,s), 8.01(1H,d,J=3.0Hz), 8.04(1H,s).

**Example 16**

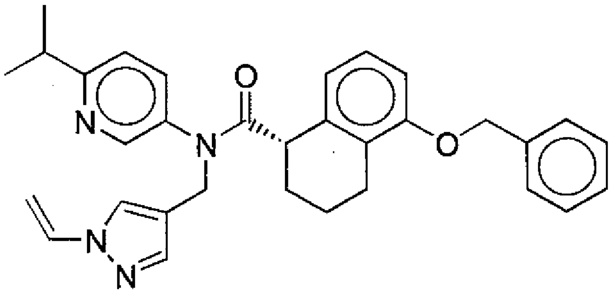

To a solution of (1S)-(-)-5-benzyloxy-1,2,3,4-tetrahydronaphthalene-1-carboxylic acid (1.14 g) in methylene chloride (10 mL) were added thionyl chloride (0.59 mL) and a catalytic amount of dimethylformamide, and the mixture was heated under reflux with stirring for 2 hr. The reaction mixture was concentrated under reduced pressure, toluene was added to the residue, and the mixture was concentrated again under reduced pressure. A solution of the residue in 1,2-dichloroethane (5 mL) was added to a solution of (6-isopropylpyridin-3-yl)[(1-vinyl-1H-pyrazol-4-yl)methyl]amine (0.98 g) and pyridine (0.64 g) in 1,2-dichloroethane (10 mL) under ice-cooling. The temperature was raised to room temperature, and the mixture was stirred at the same temperature for 5 hr. The reaction mixture was poured into 4% aqueous sodium hydrogencarbonate solution, and the mixture was partitioned and extracted with chloroform. The organic layer was washed with saturated brine, and dried over anhydrous sodium sulfate. The solvent was evaporated, and the residue was purified by silica gel column chromatography to give (1*S*)-5-benzyloxy-N-(6-isopropylpyridin-3-yl)-N-[(1-vinyl-1H-pyrazol-4-yl)methyl]-1,2,3,4-tetrahydronaphthalene-1-carboxamide (1.57 g) as brown amorphous materials.
$^1$H-NMR($CDCl_3$)$_δ$: 1.31(6H,d,J=6.9Hz), 1.41-1.60(1H,m), 1.75-2.08(3H,m), 2.63-2.83(2H,m), 3.09(1H,sept,J=6.9Hz), 3.66(1H,t,J=6.2Hz), 4.65(1H,d,J=14.4Hz), 4.81-4.93(2H,m), 5.03(2H,s), 5.43(1H,d,J=15.6Hz), 6.54(1H,d,J=7.8Hz), 6.72(1H,d,J=8.1Hz), 6.92-7.10(2H,m), 7.20(1H,d,J=8.1Hz), 7.26-7.42(6H,m), 7.48(1H,s), 7.60(1H,s), 8.42(1H,d, J=2.4Hz)
MS(ESI)m/z: 507 $[MH]^+$
optical purity 91%e.e.
analysis conditions

column: CHIRALCEL OD (0.46 $\phi\times$25 cm, DAICEL CHEMICAL INDUSTRIES, LTD.)

developing solvent: hexane/isopropanol=85/15
flow rate: 1.0 mL/min
temperature: 40°C
UV detection: 254 nm
retention time: 15.9 min.

**Example 17**

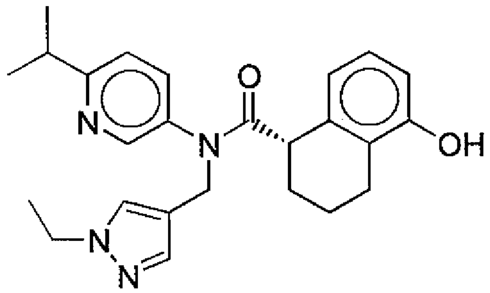

To a solution of (1*S*)-5-benzyloxy-N-(6-isopropylpyridin-3-yl)-N-[(1-vinyl-1H-pyrazol-4-yl)methyl]-1,2,3,4-tetrahydronaphthalene-1-carboxamide (1.55 g) obtained in Example 16 in ethanol (30 mL) was added 10% palladium carbon (0.4 g), and the mixture was stirred under a hydrogen atmosphere at 40°C for 4 hr. The reaction mixture was filtrated, and the solvent was evaporated. The residue was purified by silica gel column chromatography to give (1*S*)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide (1.27 g) as a white powder crystal.
$^1$H-NMR($CDCl_3$)δ: 1.31(6H,d,J=6.9Hz), 1.35-1.55(1H,m), 1.46(3H,t,J=6.9Hz), 1.75-2.10(3H,m), 2.52-2.75(2H,m), 3.10 (1H,sept,J=6.9Hz), 3.63(1H,t,J=7.2Hz), 4.14(2H,q,J=6.9Hz), 4.68(1H,d,J=14.4Hz), 4.83(1H,d,J=14.4Hz), 6.32(1H,d, J=7.8Hz), 6.39(1H,d,J=7.5Hz), 6,76(1H,t,J=7.7Hz), 6.85(1H,s), 7.21(1H,d,J=8.4Hz), 7.26-7.41(3H,m), 8.40(1H,d, J=2.4Hz)
MS (ESI)m/z: 419[MH]$^+$
optical purity 93%e.e. (analysis conditions: same as in Example 3).

**Example 18**

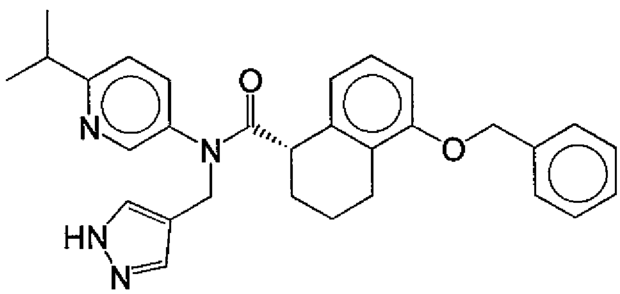

To a solution of (1*S*)-(-)-5-benzyloxy-1,2,3,4-tetrahydronaphthalene-1-carboxylic acid (0.427 g) in methylene chloride (5 mL) was added thionyl chloride (0.12 mL) and the mixture was heated under reflux with stirring for 2 hr. The reaction mixture was concentrated under reduced pressure, and a solution of the obtained residue in methylene chloride (2 mL) was added to a solution of tert-butyl 4-{[(6-isopropylpyridin-3-yl)amino]methyl}-1H-pyrazole-1-carboxylate (0.5 g), pyridine (0.18 mL) and 4-dimethylaminopyridine (4.4 mg) in methylene chloride (2 mL) under ice-cooling. The temperature was raised to room temperature, and the mixture was stirred overnight at the same temperature. The reaction mixture was poured into a saturated aqueous sodium hydrogencarbonate solution, and the mixture was partitioned and

extracted with ethyl acetate. The organic layer was washed with saturated brine, and dried over anhydrous magnesium sulfate. The solvent was evaporated, and the residue was purified by silica gel column chromatography to give tert-butyl 4-{[{[(1*S*)-5-benzyloxy-1,2,3,4-tetrahydronaphthalen-1-yl]carbonyl}(6-isopropylpyridin-3-yl)amino]methyl}-1H-pyrazole-1-carboxylate (0.673 g).
$^1$H-NMR($CDCl_3$)δ: 1.31(6H,d,J=6.9Hz), 1.40-1.70(1H,m), 1.65(9H,s), 1.75-2.10(3H,m), 2.60-2.80(2H,m), 3.10(1H,sept, J=6.9Hz), 3.60-3.70(1H,m), 4.69(1H,d,J=15.0Hz), 4.84(1H,d,J=15.0Hz), 5.03(2H,s), 6.56(1H,d,J=7.8Hz), 6.73(1H,d, J=7.8Hz), 7.06(1H,t,J=7.8Hz), 7.21(1H,d,J=8.4Hz), 7.25-7.45(6H,m), 7.67(1H,s), 7.96(1H,s), 8.41(1H,d,J=2.4Hz).

To a solution of tert-butyl 4-{[{[(1*S*)-5-benzyloxy-1,2,3,4-tetrahydronaphthalen-1-yl]carbonyl}(6-isopropylpyridin-3-yl)amino]methyl}-1H-pyrazole-1-carboxylate (0.673 g) obtained by the above-mentioned operation in dioxane (2 mL) was added 4 mol/L HCl/dioxane (5.6 mL), and the mixture was stirred at room temperature. After confirmation of completion of the reaction, the reaction mixture was poured into saturated aqueous sodium hydrogencarbonate solution, and the mixture was partitioned and extracted with ethyl acetate. The organic layer was washed with saturated brine, and dried over anhydrous magnesium sulfate. The solvent was evaporated, and the residue was purified by silica gel column chromatography to give (1*S*)-5-benzyloxy-N-(6-isopropylpyridin-3-yl)-N-[(1H-pyrazol-4-yl)methyl]-1,2,3,4-tetrahydronaphthalene-1-carboxamide (0.52 g).
$^1$H-NMR($CDCl_3$)δ: 1.31(6H,d,J=6.9Hz), 1.40-1.50(1H,m), 1.75-2.10(3H,m), 2.60-2.80(2H,m), 3.09(1H,sept,J=6.9Hz), 3.60-3.70(1H,m), 4.71(1H,d,J=14.4Hz), 4.88(1H,d,J=14.4Hz), 5.03(2H,s), 6.55(1H,d,J=7.8Hz), 6.72(1H,d,J=8.4Hz), 7.04(1H,t,J=7.8Hz), 7.20(1H,d,J=8.4Hz), 7.25-7.45(6H,m), 7.51(2H,s), 8.38(1H,d,J=2.1Hz)
MS(ESI)m/z: 481 [MH]$^+$
optical purity 84%e.e.
analysis conditions

column: CHIRALCEL OD (0.46 ϕ×25 cm, DAICEL CHEMICAL INDUSTRIES, LTD.)
developing solvent: hexane/isopropanol=85/15
flow rate: 1.0 mL/min
column oven temperature: 40°C
UV detection: 254 nm
retention time: 16.6 min.

## Example 19

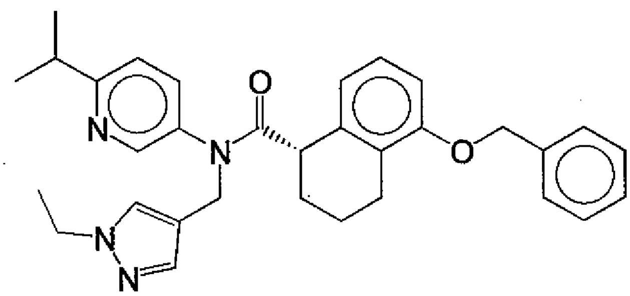

To (1*S*)-5-benzyloxy-N-(6-isopropylpyridin-3-yl)-N-E(1H-pyrazol-4-yl)methyl]-1,2,3,4-tetrahydronaphthalene-1-carboxamide (0.52 g) obtained in Example 18 were added dimethylformamide (2 mL), toluene (2 mL), ethyl iodide (0.1 mL) and potassium carbonate (0.297 g), and the mixture was stirred with heating at 50°C for 8 hr. The reaction mixture was poured into water, and the mixture was partitioned and extracted with ethyl acetate. The organic layer was washed with saturated brine, and dried over anhydrous magnesium sulfate. The solvent was evaporated, and the residue was purified by silica gel column chromatography to give -(1*S*)-5-benzyloxy-N-[(1-ethyl-1H-pyrazol-4-yl)methyl]-N (6-isopropylpyridin-3-yl)-1,2,3,4-tetrahydronaphthalene-1-carboxamide (0.287 g).
MS(ESI)m/z: 509[MH]$^+$
optical purity 81%e.e.(analysis conditions: same as in Example 3).

**Example 20**

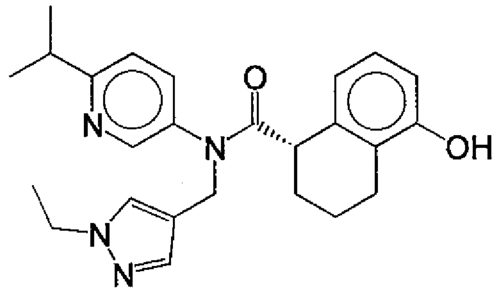

To (1*S*)-5-benzyloxy-N-[(1-ethyl-1H-pyrazol-4-yl)methyl]-N-(6-isopropylpyridin-3-yl)-1,2,3,4-tetrahydronaphthalene-1-carboxamide (0.287 g) obtained in Example 19 were added trifluoroacetic acid (1.2 mL) and thioanisole (0.2 mL), and the mixture was stirred at room temperature for 6 hr. The reaction mixture was poured into a saturated aqueous sodium hydrogencarbonate solution, and the mixture was partitioned and extracted with ethyl acetate. The organic layer was washed with saturated brine, and dried over anhydrous magnesium sulfate. The solvent was evaporated, and the residue was purified by silica gel column chromatography to give (1*S*)-(-)-N-[(1-ethyl-1H-pyrazol-4-yl)methyl]-5-hydroxy-N-(6-isopropylpyridin-3-yl)-1,2,3,4-tetrahydxonaphthalene-1-carboxamide (0.213 g).
MS(ESI)m/z: 419$[MH]^+$
optical purity 83%e.e.(analysis conditions: same as in Example 3).

**Example 21**

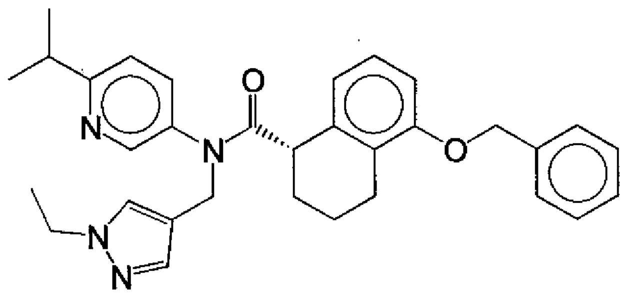

To a solution of (1*S*)-5-benzyloxy-N-(6-isopropylpyridin-3-yl)-N-[(1H-pyrazol-4-yl)methyl]-1,2,3,4-tetrahydronaphthalene-1-carboxamide (82% e.e.) (0.152 g) were added methylene chloride (1.3 mL), 1 mol/L aqueous sodium hydroxide solution (0.63 mL), ethyl iodide (0.05 mL) and tetra-n-butylammonium hydrogensulfate (0.107 g), and the mixture was stirred overnight at room temperature. The reaction mixture was poured into water, and the mixture was partitioned and extracted with ethyl acetate. The organic layer was washed with saturated brine, and dried over anhydrous magnesium sulfate. The solvent was evaporated, and the residue was purified by silica gel column chromatography to give (1*S*)-5-benzyloxy-N-[(1-ethyl-1H-pyrazol-4-yl)methyl]-N-(6-isopropylpyridin-3-yl)-1,2,3,4-tetrahydronaphthalene-1-carboxamide (0.157 g).
M5(ESI)m/z: 509$[MH]^+$
optical purity 81%e.e. (analysis conditions: same as in Example 3).

**Example 22**

Methanol (6.5 ml) was added to free base·Form-I crystal (2.0 g) and the crystals were completely dissolved by heating under reflux. The solution was allowed to cool to room temperature, and a small amount of a seed crystal was added. As a result, a white crystalline precipitate was produced. After further allowing to stand in a refrigerator overnight,

the precipitate was collected by filtration and dried under reduced pressure at 25°C for 4.5 hr to give a white solid (1.67 g).

The solid was subjected to XRD analysis under the same conditions as for free base·Form-I crystal. The XRD pattern is shown in Fig. 3. The characteristic peaks of the crystal in terms of diffraction angle 2θ were 5.9, 11.9, 15.6 and 21.3° (each ±0.2°).

The solid was subjected to DSC measurement under the same conditions as for free base·Form-I crystal. As a result, the melting point (extrapolation-onset temperature) was found at 96°C. The DSC curve is shown in Fig. 4. (Exothermic peak by crystallization into free base·Form-I crystal was observed at an extrapolation-onset temperature of 127°C and the melting peak of free base·Form-I crystal was observed at an extrapolation-onset temperature of 173°C.)

## Example 23

Acetonitrile (2 ml) was added to free base·Form-I crystal (300 mg) and the crystals were completely dissolved by heating under reflux for about 20 min. The solution was allowed to cool to room temperature, and the solvent was evaporated at 40°C. As a result, a transparent candy-like substance was obtained, which was adhered to the wall of an egg-plant flask. When the substance was further dried under reduced pressure at room temperature for 30 min, a white foamy solid was obtained. The solid part was collected to give 178 mg of a white solid (amorphous form of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide).

The solid was subjected to XRD analysis under the same conditions as for free base·Form-I crystal. The XRD pattern is shown in Fig. 5.

The solid was subjected to DSC measurement under the same conditions as for free base·Form-I crystal. As a result, the exothermic peak by crystallization into free base·Form-I crystal was observed at an extrapolation-onset temperature of 102°C and the endothermic peak due to melting of free base·Form-I crystal was observed at an extrapolation-onset temperature of 171°C. The DSC curve is shown in Fig. 6.

## Example 24

To free base·Form-I crystal (4.0 g) was added n-propanol (20 ml) and the crystals were completely dissolved by heating under reflux in an oil bath. The solution was allowed to cool to room temperature, and 2 mol/L hydrochloric acid/ethanol solution (5.7 ml) was added dropwise with stirring. A small amount of a seed crystal was added and the mixture was ice-cooled. As a result, a white crystalline precipitate was produced. After further allowing to stand in a refrigerator overnight, the precipitate was collected by filtration and dried under reduced pressure at 40°C for 4 hr to give 3.80 g of a white solid ((1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide monohydrochloride).

The results of the elemental analysis of this solid were C:65.76,H:6.86,N:12.14,Cl:7.57% (Calculated; C: 65.99,H:6.87,N:12.31,Cl:7.79%).

The solid was subjected to DSC measurement under the same conditions as for free base·Form-I crystal. As a result, melting and/or decomposition peak(s) were found at an extrapolation-onset temperature of 172°C. The DSC curve is shown in Fig. 7.

## Example 25

To free base•Form-1 crystal (2.01 g) was added acetone (7 ml) and the crystals were completely dissolved by heating under reflux in an oil bath. The solution was allowed to cool to room temperature, and 2 mol/L hydrobromide/ethanol solution (2.4 ml, 1.2 equivalent) was added dropwise with stirring. A small amount of a seed crystal was added and the mixture was ice-cooled. As a result, a white crystalline precipitate was produced. After further allowing to stand in a refrigerator overnight, the precipitate was collected by filtration and dried under reduced pressure at 40°C for 2 hr to give 1.59 g of a white solid ((1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide monohydrobromide). The results of the XRD of the compound obtained here matched with those obtained in Example 27. $^1$H-NMR(DMSO-$d_6$)δ: 1.15-1.50(10H,m), 1.70-2.00(3H,m), 2.35-2.60(2H, m), 3.15(1H,brs), 3.51(1H,brs), 4.06(2H,q,J=7.3Hz), 4.50-4.85(2H,m), 6.45(1H,d,J=7.7Hz), 6.61(1H,d,J=7.9Hz), 6.88 (1H,t,J=7.8Hz), 7.24(1H,brs), 7.45-7.70(2H,m), 7.91(1H,brs), 8.58(1H,brs)

The solid was subjected to DSC measurement under the same conditions as for free base · Form-I crystal. As a result, melting and/or decomposition peak(s) were found at an extrapolation-onset temperature of 189°C. The DSC curve is shown in Fig. 8.

## Example 26

To free base·Form-I crystal (2.00 g) was added methanol (6.5 ml) and the crystals were completely dissolved

by heating under reflux. The solution was allowed to cool to room temperature, and a small amount of a seed crystal was added. As a result, a white crystalline precipitate was produced. After further allowing to stand in a refrigerator overnight, the precipitate ((1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide monomethanol solvate) was collected by filtration. A small amount of the solid was taken, gently dried with filter paper, and subjected to XRD analysis while wet under the same conditions as for free base·Form-I crystal. As a result, a pattern similar to the XRD pattern of the compound obtained in Example 22 was obtained. The XRD pattern is shown in Fig. 9. The characteristic peaks of the crystal in terms of diffraction angle 2θ were 6.0, 12.0, 18.0 and 21.9° (each ±0.2°). The difference from the XRD pattern of the compound obtained in Example 22 includes the fact that the monomethanol solvate has a peak at 21.9°. Other difference includes the fact that the monomethanol solvate showed a broad peak near 20.7 - 20.9° (each ±0.2°), while the compound of Example 22 showed a peak at 21.3° (±0.2°).

[Preparation of samples for NMR measurement]

The compound obtained in Example 22 was further dried under reduced pressure at 40°C for 35 min, placed flat in a weighting bottle (about 30 mg) free of a lid, and preserved in a methanol desiccator (brown) at room temperature. The compound was taken out after 8 days of preservation, and subjected to the NMR measurement. The peaks considered to have derived from methanol were found at 3.17 and 4.10 ppm. In addition, a sample after drying under reduced pressure at 40°C for 35 min was subjected to XRD analysis under the same conditions as for free base · Form-I crystal. As a result, the pattern matched with the XRD pattern of the compound of Example 22. The results are shown in Fig. 10.
$^1$H-NMR(DMSO-$d_6$)δ: 1.22(6H,d,J=6.9Hz), 1.25-1.45(1H,m), 1.30(3H,t,J=7.3Hz), 1.70-2.00(3H,m), 2.35-2.55(2H,m), 3.03(1H,sept,J=6.9Hz), 3.17(3H,d,J=5.3Hz), 3.46(1H,t,J=6.8Hz), 4.06(2H,q,J=7.3Hz), 4.10(1H,q,J=5.3Hz), 4.66(1H,d, J=14.8Hz), 4.71(1H,d,J=14.8Hz), 6.42(1H,d,J=7.6Hz), 6.60(1H,d,J=7.9Hz), 6.88(1H,t,J=7.8Hz), 7.21(1H,s), 7.36(1H,d, J=8.2Hz), 7.51(1H,s), 7.63(1H,dd,J=2.2Hz,8.2Hz), 8.38(1H,d,J=2.2Hz), 9.22(1H,s).

**Example 27**

To free base·Form-I crystal (100 mg) was added dropwise 2 mol/l hydrobromic acid/ethanol solution (143 μl, 1.2 equivalent). After dispersing by ultrasonication, the mixture was heated to 60°C, and acetone (1 ml) was added dropwise. A small amount of a seed crystal was added and the mixture was allowed to stand at room temperature. As a result, a white solid was precipitated. After allowing to stand at room temperature for about 4.5 hr and further in a refrigerator overnight, the precipitate was collected by filtration and dried under reduced pressure at 40°C for 3 hr to give 62 mg of a white solid ((1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide monohydrobromide). The solid was subjected to an elemental analysis and found to show C: 59.99,H:6.14,N:10.96,Br:15.74 (Calculated;
C:60.12,H:6.26,N:11.22,Br:16.00%).

**Preparation Example 5**

5-Benzyloxy-1,2,3,4-tetrahydronaphthalene-1-carboxylic acid (230.0 g) and (R)-(+)-1-phenylethylamine (98.7 g) were dissolved in THF (575 mL), and cooled to 10°C or below to give crude crystals (138.9 g). The crystals were recrystallized from THF to give (R)-(+)-1-phenylethylammonium (1S)-(-)-5-benzyloxy-1,2,3,4-tetrahydronaphthalene-1-carboxylate (105.0 g) as white crystals. The crystals (100 g) were dissolved in ethanol (200 mL) and water (50.0 mL). Aqueous hydrochloric acid prepared from concentrated hydrochloric acid (56.8 g) and water (350 mL) was added and the crystals were collected by filtration to give (1S)-(-)-5-benzyloxy-1,2,3,4-tetrahydronaphthalene-1-carboxylic acid (60.4 g) as pale-yellow brown crystals.

**Preparation Example 6**

To 1-ethyl-1H-pyrazole-4-carboxylic acid (12.68 g) were added toluene (56.0 mL) and dimethylformamide (112 μL), the temperature was raised to 62°C, and thionyl chloride (9.90 mL) was added dropwise. The mixture was stirred at 66°C for 1.5 hr, concentrated, and purified by distillation to give 1-ethyl-1H-pyrazole-4-carboxylic acid chloride (12.57 g) as a colorless transparent oil.
$^1$H-NMR($CDCl_3$)δ: 1.55(3H,t,J=7.3Hz), 4.22(2H,q,J=7.4Hz), 7.99(1H,s), 8.02 (1H,s)

**Preparation Example 7**

To (1S)-(-)-5-benzyloxy-1,2,3,4-tetrahydronaphthalene-1-carboxylic acid (1.00 g) were added toluene (5.0 mL)

and dimethylformamide (0.01 mL), and thionyl chloride (0.31 mL) was added dropwise. The mixture was stirred at 45°C for 1 hr and concentrated to give (1S)-(-)-5-benzyloxy-1,2,3,4-tetrahydronaphthalene-1-carboxylic acid chloride (1.10 g) as a pale-brown oil.

$^1$H-NMR($CDCl_3$)δ: 1.81-1.95(2H,m), 2.09-2.18(1H,m), 2.31-2.39(1H,m), 2.78(2H,dtd,J=5.9, 12.2,44.6Hz), 4.25(1H,t, J=6.0Hz), 5.07(2H,s), 6.82(2H,dd,J=7.9,21.8Hz), 7.13-7.44(6H,m).

**Preparation Example 8**

A suspension of 2-hydroxy-6-isopropylnicotinonitrile (264 g) and phosphorus oxychloride (500 g) was dissolved by heating under stirring, and refluxed for 2 hr. After cooling, the reaction solution was poured into ice water (3 1), and the mixture was stirred for a while and partitioned and extracted with toluene. The organic layer was washed with saturated brine, and dried over magnesium sulfate. The solvent was evaporated to give 2-chloro-6-isopropylnicotinonitrile (313.6 g) as a brown oil.

$^1$H-NMR($CDCl_3$)δ: 1.32(6H,d,J=6.9Hz), 3.12(1H,sept,J=6.9Hz), 7.28(1H,d,J=8.1Hz), 7.94(1H,d,J=8.1Hz).

**Preparation Example 9**

2-Chloro-6-isopropylnicotinonitrile (313.6 g) and 75% sulfuric acid (800 mL) were stirred at an inside temperature of 110°C for 2.5 hr. After cooling, the reaction solution was poured into ice water (4 L), and the mixture was stirred for a while and partitioned and extracted with toluene. The organic layer was washed with saturated brine, and dried over magnesium sulfate. The solvent was evaporated and, after seeding, the precipitated solid was suspended in hexane and a small amount of IPE and collected by filtration to give 2-chloro-6-isopropylnicotinic acid (285 g) as brown powder crystals.

$^1$H-NMR($CDCl_3$)δ: 1.31(6H,d,J=6.9Hz), 3.13(1H,sept,J=6.9Hz), 7.20(1H,d,J=8.1Hz), 8.29(1H,d,J=8.1Hz), 12.40(1H, brs).

**Preparation Example 10**

2-Chloro-6-isopropylnicotinic acid (312 g), palladium carbon(10%) (16.0 g) and ethanol-water (6:1) (2000 mL) were stirred under a hydrogen atmosphere at room temperature for 3 days. The reaction solution was filtrated, and the filtrate was concentrated under reduced pressure. Ethyl acetate and a small amount of ethanol were added to the residue, and the precipitated solid was collected by filtration. The solid was added to cooled 1N-aqueous sodium hydroxide solution (1130 mL) and, after dissolution, the precipitated solid was collected by filtration. This was dissolved in chloroform, and dried over magnesium sulfate. The solvent was evaporated, the residual solid was suspended in hexane and collected by filtration to give 6-isopropylnicotinic acid (152 g) as white powder crystals.

$^1$H-NMR($CDCl_3$)δ: 1.37(6H,d,J=6.9Hz), 3.29(1H,sept,J=6.9Hz), 7.38(1H,d,J=8.1Hz), 8.40(1H,dd,J=2.1,8.1Hz), 9.33 (1H,d,J=2.1Hz).

**Preparation Example 11**

To a solution of 6-isopropylnicotinic acid (148 g) and triethylamine (250 mL) in tert-butanol (1800 mL) were added dropwise diphenylphosphoryl azide (212 mL) at room temperature, and the mixture was heated under reflux with stirring for 2 hr. The reaction mixture was concentrated under reduced pressure. Water was added to the residue, and the mixture was partitioned and extracted with ethyl acetate. The organic layer was concentrated under reduced pressure, the residual solid was suspended in water and collected by filtration to give tert-butyl(6-isopropylpyridin-3-yl)carbamate (137 g) as pale-yellow powder crystals.

$^1$H-NMR($CDCl_3$)δ: 1.27(6H,d,J=6.9Hz), 1.51(9H,s), 3.03(1H,sept,J=6.9Hz), 6.90-7.03(1H,m), 7.12(1H,d,J=8.4Hz), 7.85-8.01(1H,brs), 8.35(1H,d,J=2.4Hz).

**Preparation Example 12**

To tert-butyl(6-isopropylpyridin-3-yl)carbamate (50.0 g) was added 4 mol/L-hydrochloric acid/dioxane (432 mL), and the mixture was stirred at room temperature for one day. To the reaction mixture containing the precipitated solid was added diethyl ether (500 mL) and the solid was collected by filtration. The solid was added to an aqueous sodium hydrogencarbonate solution, and the mixture was partitioned and extracted with diethyl ether. The organic layer was washed with saturated brine, and dried over sodium sulfate. The solvent was evaporated to give 6-isopropylpyridine-3-amine (26.4 g) as a brown oil.

$^1$H-NMR($CDCl_3$)δ: 1.26(6H,d,J=6.9Hz), 2.96(1H,sept,J=6.9Hz), 3.35-3.68(2H,brs), 6.95(2H,m), 8.03(1H,m)

The "racemate of the compound of Example 3" in the following Experimental Examples 1 - 3 and 5, and the "racemate" in Experimental Example 4 were obtained according to the method described in Example 89 of WO02/22556.

**Experimental Example 1:** C5a receptor binding assay

The C5a receptor binding inhibitory action of C5a and the test compound was evaluated by a receptor binding assay comprising of human peripheral blood neutrophil, which expresses the C5a receptor, and [$^{125}$I]-human C5a (Amersham Pharmacia Biotech) in a MultiScreen (MILLIPORE). First, neutrophil fraction was separated from human peripheral intravenous blood using lympholyte-poly (Cedarlane), and suspended in a Binding buffer [50 mM HEPES, 1 mM $CaCl_2$, 5 mM $MgCl_2$, 0.5% bovine albumin (BSA, SIGMA), 0.02% $NaN_3$ (pH 7.2)]. The binding assay was started by the addition of $1 \times 10^5$ cells/50 μL neutrophil suspension, 25 μL of a test compound solution (obtained by dissolving the test compound in N,N-dimethylformamide to a final concentration of 10 mmol/L and diluting with binding buffer), and 25 μL of [$^{125}$I]-C5a solution (final concentration 200 pM), to each well of the MultiScreen. For calculation of specific binding, wells containing a non-labeled C5a (final concentration 20 nM) or Binding Buffer instead of the test compound solution were prepared. After incubation at 4°C for 2 hr, suction filtration and addition of 300 μL of the Binding buffer were repeated 4 times to remove non-binding portion. After drying the MultiScreen, the radioactivity on the filter was measured using a gamma counter.
The rate of inhibition (% inhibition) of C5a binding by the test compound was calculated by the following formula using the count value obtained without addition of the test compound as Total, the count value obtained with addition of non-labeled C5a as Non, and the count value obtained with addition of the test compound as Test.

$$\%\ \text{Inhibition} = 100-[(\text{Test}-\text{Non})/(\text{Total}-\text{Non})]\times 100$$

Further, the concentration ($IC_{50}$ value) of the test compound, at which binding of [$^{125}$I]-human C5a is inhibited by 50%, was calculated by two-interpolation method. In this evaluation system, the $IC_{50}$ value of the compound of Example 3 was 10nmol/L, and the $IC_{50}$ value of the racemate of the compound of Example 3 was 23 nmol/L.

**Experimental Example 2:** Action of C5a-stimulated neutrophil on production of reactive oxygen species

A neutrophil fraction was separately taken from human peripheral venous blood using Lympholyte-poly (Cedarlane), and suspended in Hank's Balanced Salt Solution (HBSS, GIBCO BRL) containing 1% fetal bovine serum (FBS) and 1 mmol/L luminol (Wako Pure Chemical Industries, Ltd.). Reactive oxygen species was measured using a luminometer (MicroLumat, Berthold) for 96-well plate. That is, $1\times10^5$ cells/150 μL neutrophil suspension and 25 μL of a test compound solution (obtained by dissolving the test compound in N,N-dimethylformamide to a final concentration of 10 mmol/L and diluting with HBSS supplemented with 1% FBS) were added to a well, which was set in a MicroLumat set for 37°C and stood for about 5 min. Then, 25 μL of C5a (final concentration 3 nmol/L) was added and luminescence produced by the reaction of the luminol and the reactive oxygen species was measured with the lapse of time for 15 min. The rate of inhibition (% inhibition) of the production of reactive oxygen species in C5a stimulated neutrophil by the test compound was calculated by the following formula, wherein the peak value of the production of reactive oxygen species derived by C5a without addition of the test compound is Max, the peak value of the production of reactive oxygen species without addition of the test compound and without C5a stimulation is Min, and the peak value of the production of reactive oxygen species derived by C5a with the addition of the test compound is Test.

$$\%\ \text{Inhibition} = 100-[(\text{Test}-\text{Min})/(\text{Max}-\text{Min})]\times 100$$

In addition, the concentration of the test compound, at which the production of reactive oxygen species in C5a stimulated neutrophil is inhibited by 50% ($IC_{50}$ value), was calculated by two-interpolation method.
The $IC_{50}$ value of the compound of Example 3 was 2.8 nmol/L, and the $IC_{50}$ value of the racemate of the compound of Example 3 was 5.9 nmol/L.

**Experimental Example 3:** Action on collagen-induced arthritis in monkey

An emulsion of type II collagen derived from bovine (purchased from Collagen Research Center) and complete Freund's adjuvant H37Rv (purchased from Becton, Dickinson and Company) was intradermally inoculated twice to the back of cynomolgus monkey on the first day (day 0) and day 21 of testing. The monkeys that developed arthritis were

selected, divided into groups of 10 monkeys per group, and the test compound or a control medium was orally administered from day 36 to day 50. Arthritis was evaluated according to the articular swelling score. That is, the level of swelling of the joint of finger (56 joints of fingers/monkey) and respective joints of elbow, knee, wrist and ankle (total 64 joints/monkey) was scored (0 - 3), and the total of the scores for each monkey was calculated and used as the articular swelling score of each monkey. The definition of the score is as follows.
Score 0: Normal.
Score 1: Swelling can be confirmed by palpation.
Score 2: Swelling was suspected by visual observation, and swelling can be confirmed by palpation.
Score 3: Swelling can be confirmed by visual observation alone. The articular swelling scores before administration were control group: average 49, and Example 3 compound group: average 49, with no difference between the two groups. However, the articular swelling scores at day 14 of administration were control group: average 82, and Example 3 compound group: average 61, and the compound of Example 3 significantly suppressed aggravation of arthritis.

**Experimental Example 4:** evaluation of biological availability (bioavailability) in rat

Free base·Form-I crystal was administered to female SD rats fasted from the day before the experiment and the plasma concentration was measured under the following conditions. As a result, the areas under blood concentration-time curve (AUC) of the drug after intravenous administration and oral administration are as shown in Table 1. The biological availability (%) was calculated based on AUC (oral)/AUC (intravenous)×dose (oral)×dose (intravenous)×100 and found to be 62%.
Table 1

| | AUC (ng•h/mL) |
|---|---|
| Intravenous administration (1 mg/kg) | 263.97 |
| Oral administration (10 mg/kg) | 1641.43 |

I. Drug administration

1) Intravenous administration

Dosing formulation: 0.5 mg/ml in aqueous physiological saline. Dose: 1 mg/2 ml/kg
Administration method: bolus administration from the tail vein Collection of blood samples: About 0.1 - 0.2 mL of blood was taken from the cervical vein at 3 min, 10 min, 30 min, 1 hr, 2 hr, 4 hr and 6 hr after administration. The blood was
centrifuged, and the plasma was separated.

2) Oral administration

Dosing formulation: 2 mg/ml suspension adjusted by gradually adding aqueous hydroxypropylmethylcellulose solution to the compound of the present invention thoroughly triturated in an
agate mortar. Dose: 10 mg/5 ml/kg
Administration method: Forcible administration into the stomach Collection of blood samples: About 0.1 - 0.2 mL of blood was
taken from the cervical vein at 30 min, 1 hr, 2 hr, 4 hr, 6 hr, 8 hr and 24 hr after administration. The blood was centrifuged, and the plasma was separated.

II. Measurement of concentration

The plasma concentration of the administered compound was measured by LC-MS/MS.
LC conditions

Column: Capcellpak C18 UG-120 (2.0 ϕmm×50 mm, S-5 μm, Shiseido Co., Ltd.)
Mobile phase: acetonitrile
Flow rate: 0.25 mL/min
Column temperature: room temperature
Auto sampler temperature set to: 25°C

Gradient setting:
0 min→0. 6 min:25%→25%
0.6 min→3.6 min:25%→75%
3.6 min→6 min:75%→75%
6 min→6.1 min:75%→25%
6.1 min→9.5 min:25%→25%
MS/MS conditions
Ionization method: ESI
Capillary temperature: 350°C
Sheath gas pressure: 80 psi
Spray voltage: 4.5 kV.

The plasma concentration of the racemate was measured by a similar method, and the biological availability was calculated and found to be 20%. The AUC of the racemate is shown in Table 2.

Table 2

| | AUC (ng•h/mL) |
|---|---|
| Intravenous administration (1 mg/kg) | 288.6 |
| Oral administration (10 mg/kg) | 572.4 |

**Experimental Example 5:** Dissolution test

The compounds obtained in Examples 22, 24 and 25, free base·Form-I crystal, and the racemate of the compound of Example 3 were subjected to a dissolution test, and the dissolution rate was compared. According to this test method, the amount of dissolution relative to the content of each compound in the test solution is expressed in the dissolution rate (%), which was measured over time.

For the method of adding each compound, the compound (about 10 mg based on free form) was precisely weighed and directly added to a vessel. As the test solution, The Japanese Pharmacopoeia second fluid (900 ml) was used, and the test was performed according to The Japanese Pharmacopoeia Dissolution Test Method 2 (Paddle Method) at 50 rpm. The test temperature was 37°C. After the start of the test, solution for dissolution (10 ml) was taken at suitable times, and a test solution (10 ml) heated to 37°C was soon carefully added for compensation. The collected solution for dissolution was passed through a filter (DISMIC 13HP PTFE (0.45 μm); Toyo Roshi Kaisha). The filtrate in the early stage (5 ml) was removed, the subsequent filtrate (500 μl) was precisely measured, and the dilution solution (water/acetonitrile/trifluoroacetic acid mixture=500:500:1, 500 μl) was accurately added to give a sample solution.

Separately, about 10 mg of the free base·Form-I crystal was precisely weighed, and a dilution solution (water/acetonitrile/trifluoroacetic acid mixture=500:500:1) was added to 50 ml sharp. This solution (2.5 ml) was precisely measured, and a dilution solution (water/acetonitrile/trifluoroacetic acid mixture=500:500:1) was added to 50 ml sharp. This was used as the standard solution of free base·Form-I crystal (10 μg/ml). The sample solution and the standard solution were measured under the HPLC conditions shown below, the peak areas $A_T$ and $A_S$ of the free base·Form-I crystal in each solution were measured, and the dissolution rate was determined by the following formula. The results are shown in Fig. 11.

The dissolution rate of the racemate was the slowest, then in the order of the free base·Form-I crystal, and the compounds of Example 25, Example 24 and Example 22. From these results, the dissolution rate of the compounds of Examples 22, 24 and 25 was faster than that of the free base • Form-I crystal, and the compounds are considered to be rapidly dissolved and absorbed in living body. Therefore, the biological availability of the compounds of Examples 22, 24 and 25 is considered to be higher than that of the free base·Form-I crystal.

[Calculation method of dissolution rate]

The nth dissolution rate (%) of free base·Form-I

$$\text{crystal} = W_n / W_{100\%} \times 100$$

$W_1$: the first dissolution amount (mg) of free base • Form-I crystal

$$=(900 \times A_{T1}) \times W_s/A_s/1000$$

$W_n$: the nth (n>1) dissolution amount (mg) of free base • Form-I crystal

$$=\{900 \times A_{Tn}+10 \times (A_{T1}+A_{T2}...+A_{Tn-1})\} \times W_s/A_s/1000$$

$W_{100\%}$ : cast amount (mg) of drug substance
$W_s$ : collected amount (mg) of free base · Form-I crystal standard product
$A_s$ : peak area of free base·form-I crystal in standard solution
$A_{T1}$ : peak area of free base·Form-I crystal in sample solution by the first sampling
$A_{Tn}$ : peak area of free base·form-I crystal in sample solution by the nth sampling.

For racemate, the dissolution rate was calculated based on the total value of S form and R form.

[HPLC conditions]

Detection wavelength: 221 nm
Column: Inertsil ODS-3V (4.6 $mm_{\phi} \times$150 mm)
Column temperature: 40°C
Mobile phase: A; 0.1% trifluoroacetic acid solution B; 0.1% trifluoroacetic acid/acetonitrile solution A:B=72:27 (isocratic elution)
Flow rate: 1.0 ml/min.

**Experimental Example 6:** Solubility test

An adequate amount of a sample was taken, and a test solution (The Japanese Pharmacopoeia Solution 2) was added to each to a concentration of about 1 mg/ml. The sample was dispersed by ultrasonication for 2 min, shaken at 37°C for 30 min, and the solution was filtered through DISMIC13HP (0.45 mm). The filtrate in the early stage (3 ml) was removed, the subsequent filtrate (500 μl) was precisely measured, and the dilution solution (water/acetonitrile/trifluoroacetic acid mixture=500:500:1, 500 μl) was accurately added to give a sample solution. The sample solution and the standard solution prepared in Experimental Example 5 were subjected to the measurement under the similar HPLC conditions as in Experimental Example 5.
The solubility after 30 min was 165 μg/ml for the compound obtained in Example 23, 66 μg/ml for the free base·Form-I crystal, and 31 μg/ml for the racemate of the compound of Example 3.
The solubility of the compound obtained in Example 23 (amorphous form) was 2.5-fold of free base·Form-I crystal and about 5-fold of the racemate, and the compound is considered to be superior as a drug substance for pharmaceutical products.

**Industrial Applicability**

The compound of the present invention, (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof, a hydrate thereof and a solvate thereof show not only a C5a receptor antagonistic activity but also high activity in the biological availability, as compared to its racemate.
This application has been filed claiming the priority benefit based on a Japanese patent application No. 2005-29907.

## Claims

1. (1S)-(-)-N-[(1-Ethyl-1H-pyrazol-4-yl)methyl]-5-hydroxy-N-(6-isopxopylpyridin-3-yl)-1,2,3,4-tetrahydronaphthalene-1-carboxamide represented by the following formula (I)

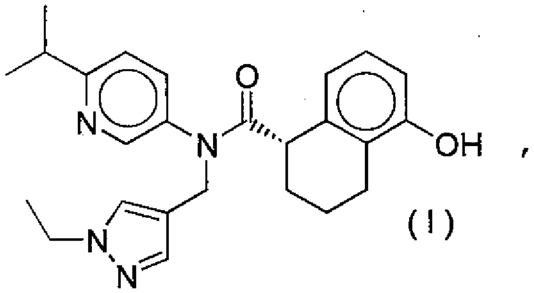

(I)

a pharmacologically acceptable salt thereof or a hydrate thereof or a solvate thereof.

2. A crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.

3. The crystal of claim 2, which is a crystal of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.

4. The crystal of claim 2 or 3, which shows a peak at a diffraction angle 2θ of about 14.6° (±0.2°) in powder X-ray diffraction spectrum.

5. The crystal of any one of claims 2 to 4, which shows a peak at a diffraction angle 2θ of about 10.0° (±0.2°) in powder X-ray diffraction spectrum.

6. The crystal of any one of claims 2 to 5, which shows a peak at a diffraction angle 2θ of about 8.5° (±0.2°) in powder X-ray diffraction spectrum.

7. The crystal of any one of claims 2 to 6, which shows peaks at a diffraction angle 2θ of about 20.1 and 23.2° (±0.2°, respectively) in powder X-ray diffraction spectrum.

8. The crystal of any one of claims 2 to 7, which shows characteristic peaks at a diffraction angle 2θ of about 8.5, 10.0, 14.6, 20.1 and 23.2° (each ±0.2°) in powder X-ray diffraction spectrum.

9. The crystal of any one of claims 2 to 8, which has a melting point (extrapolation-onset temperature) of about 171 to about 176°C.

10. The crystal of any one of claims 2 to 9, which has a melting point (extrapolation-onset temperature) of about 176°C.

11. The crystal of any one of claims 2 to 10, which has the physicochemical property shown in the following A and/or B:

    A: having a powder X-ray diffraction pattern shown in Fig. 1
    B: having a differential scanning calorimetry curve shown in Fig. 2.

12. The crystal of claim 2 or 3, which shows a peak at a diffraction angle 2θ of about 5.9° (±0.2°) in powder X-ray diffraction spectrum.

13. The crystal of claim 2, 3 or 12, which shows a peak at a diffraction angle 2θ of about 15.6° (±0.2°) in powder X-ray diffraction spectrum.

14. The crystal of claim 2, 3, 12 or 13, which shows a peak at a diffraction angle 2θ of about 11.9° (±0.2°) in powder X-ray diffraction spectrum.

15. The crystal of claim 2, 3, 12, 13 or 14, which shows a peak at a diffraction angle 2θ of about 21.3° (±0.2°) in powder X-ray diffraction spectrum.

16. The crystal of claim 2, 3, 12, 13, 14 or 15, which shows characteristic peaks at a diffraction angle 2θ of about 5.9, 11.9, 15.6 and 21.3° (±0.2°, respectively) in powder X-ray diffraction spectrum.

17. The crystal of claim 2, 3, 12, 13, 14, 15 or 16, which has a melting point (extrapolation-onset temperature) of about 96°C.

18. The crystal of claim 2, 3, 12, 13, 14, 15, 16 or 17, which has the physicochemical property shown in the following C and/or D:

C: having a powder X-ray diffraction pattern shown in Fig. 3
D: having a differential scanning calorimetry curve shown in Fig. 4.

19. An amorphous form of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.

20. The amorphous form of claim 19, which is an amorphous form of (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.

21. The amorphous form of claim 19 or 20, which has the physicochemical property shown in the following E:

E: having a powder X-ray diffraction pattern shown in Fig. 5.

22. The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1, whose pharmacologically acceptable salt is a hydrochloride.

23. The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1 or 22, whose pharmacologically acceptable salt is a monohydrochloride.

24. The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1, whose pharmacologically acceptable salt is a hydrobromide.

25. The (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1 or 24, whose pharmacologically acceptable salt is a monohydrobromide.

26. A pharmaceutical agent comprising the compound of any one of claims 1 to 25.

27. A pharmaceutical composition comprising the compound of any one of claims 1 to 25, and a pharmaceutically acceptable additive.

28. A drug for the prophylaxis or treatment of a disease caused by binding of C5a with a C5a receptor, which comprises the compound of any one of claims 1 to 25 as an active ingredient.

29. The drug of claim 28, wherein the disease caused by the binding of C5a with a C5a receptor is autoimmune disease, sepsis, adult respiratory distress syndrome, chronic obstructive pulmonary disease, allergic disease, atherosclerosis, myocardial infarction, cerebral infarction, psoriasis, Alzheimer's disease, or organ injury caused by leukocyte activation due to ischemia reperfusion, trauma, burn or surgical invasion.

30. An anti-inflammatory agent comprising the compound of any one of claims 1 to 25 as an active ingredient.

31. A C5a receptor antagonist comprising the compound of any one of claims 1 to 25 as an active ingredient.

**32.** The antagonist of claim 31, which is a drug for the prophylaxis and/or treatment of infection with bacterium or virus that invades via a C5a receptor.

**33.** A drug for the prophylaxis and/or treatment of rheumatoid arthritis, which comprises, as an active ingredient, (1S)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof.

**34.** The drug of claim 33, wherein the active ingredient is (IS)-(-)-N-[(1-ethyl-1H-pyrazol-4-yl)methyl]-5-hydroxy-N-(6-isopropylpyridin-3-yl)-1,2,3,4-tetrahydronaphthalene-1-carboxamide.

**35.** The drug of claim 33 or 34, wherein the active ingredient is a crystal of (1S)-(-)-N-[(1-ethyl-1H-pyrazol-4-yl)methyl]-5-hydroxy-N-(6-isopropylpyridin-3-y1)-1,2,3,4-tetrahydronaphthalene-1-carboxamide.

**36.** The drug of any one of claims 33 to 35, wherein the active ingredient shows characteristic peaks at a diffraction angle 2θ of about 8.5, 10.0, 14.6, 20.1 and 23.2° (each ±0.2°) in powder X-ray diffraction spectrum.

**37.** The drug of any one of claims 33 to 36, wherein the active ingredient has a melting point (extrapolation-onset temperature) of about 176°C.

**38.** 1-Ethyl-N-(6-isopropylpyridin-3-yl)-1H-pyrazole-4-carboxamide or N-(6-isopropylpyridin-3-yl)-1-vinyl-1H-pyrazole-4-carboxamide, which is represented by the following formula (II)

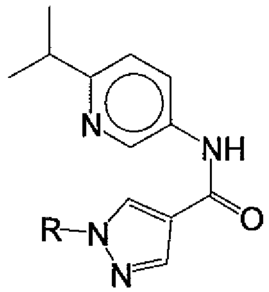

(II)

wherein R is ethyl or vinyl.

**39.** [(1-Ethyl-1H-pyrazol4-yl)methyl](6-isopropylpyridin-3-yl)amine or (6-isopropylpyridin-3-yl)[(1-vinyl-1H-pyrazol-4-yl)methyl]amine, which is represented by the following formula (III)

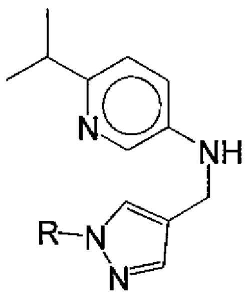

(III)

wherein R is ethyl or vinyl.

**40.** A 1-ethyl-1H-pyrazole compound represented by the following formula (IV)

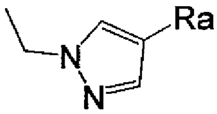

(IV)

wherein Ra is hydroxymethyl or formyl.

**41.** Ethyl 1-(2-chloroethyl)-1H-pyrazole-4-carboxylate represented by the following formula (V)

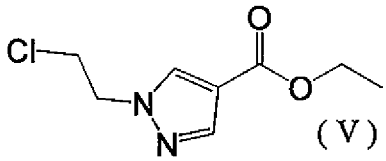

(V)

**42.** A 1-vinyl-1H-pyrazole compound represented by the following formula (VI)

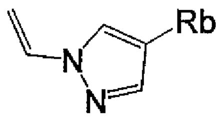

(VI)

wherein Rb is ethoxycarbonyl, hydroxycarbonyl, hydroxymethyl or formyl.

**43.** (1S)-(-)-N-[(1-Ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1, wherein the solvate is a methanol solvate.

**44.** (1S)-(-)-N-[(1-Ethyl-1H-pyrazol-4-yl)methyl]-5-hydroxy-N-(6-isopropylpyridin-3-yl)-1,2,3,4-tetrahydronaphthalene-1-carboxamide, a pharmacologically acceptable salt thereof or a hydrate thereof or a solvate thereof of claim 1 or 43, wherein the solvate is a monomethanol solvate.

FIG. 1

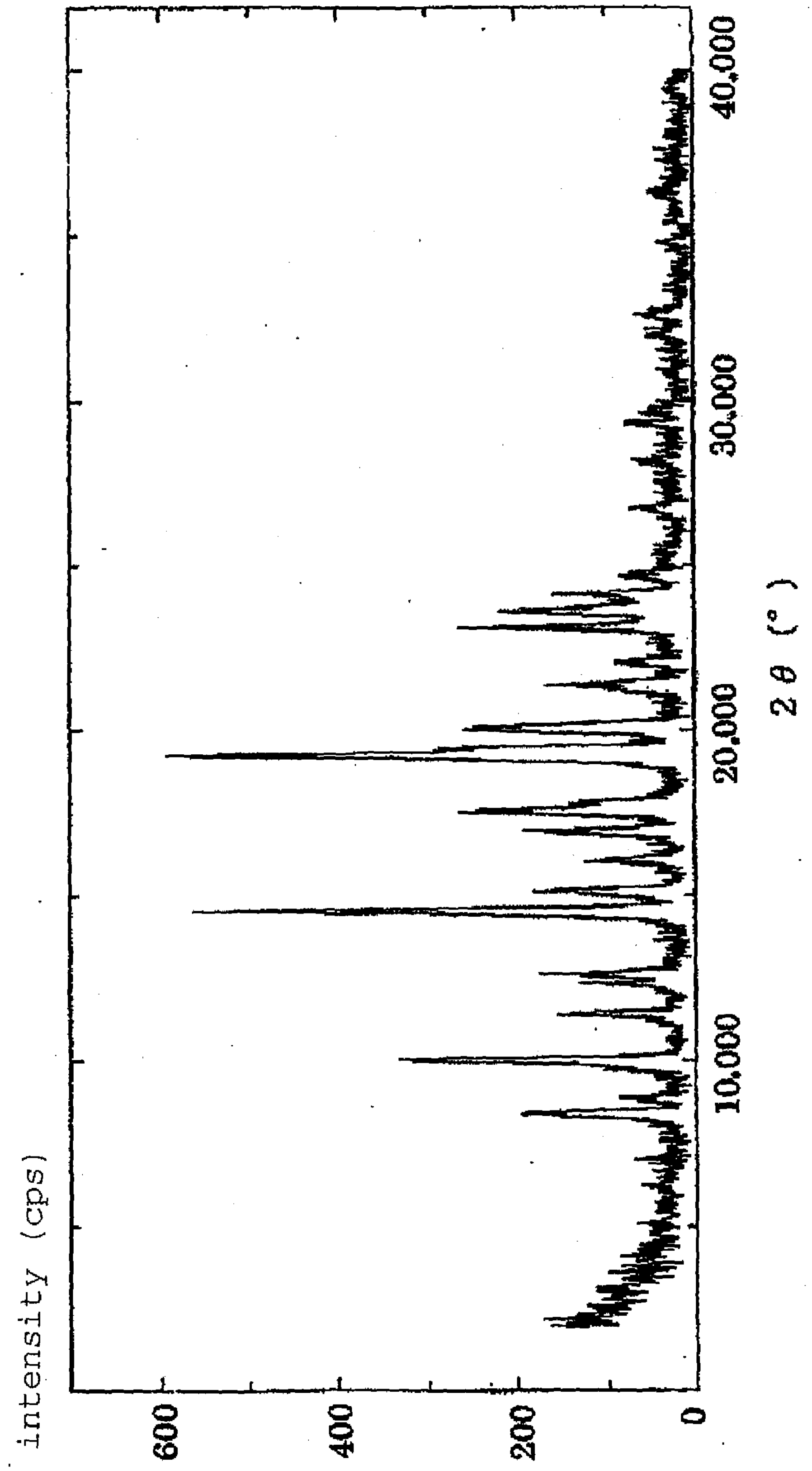

FIG. 2

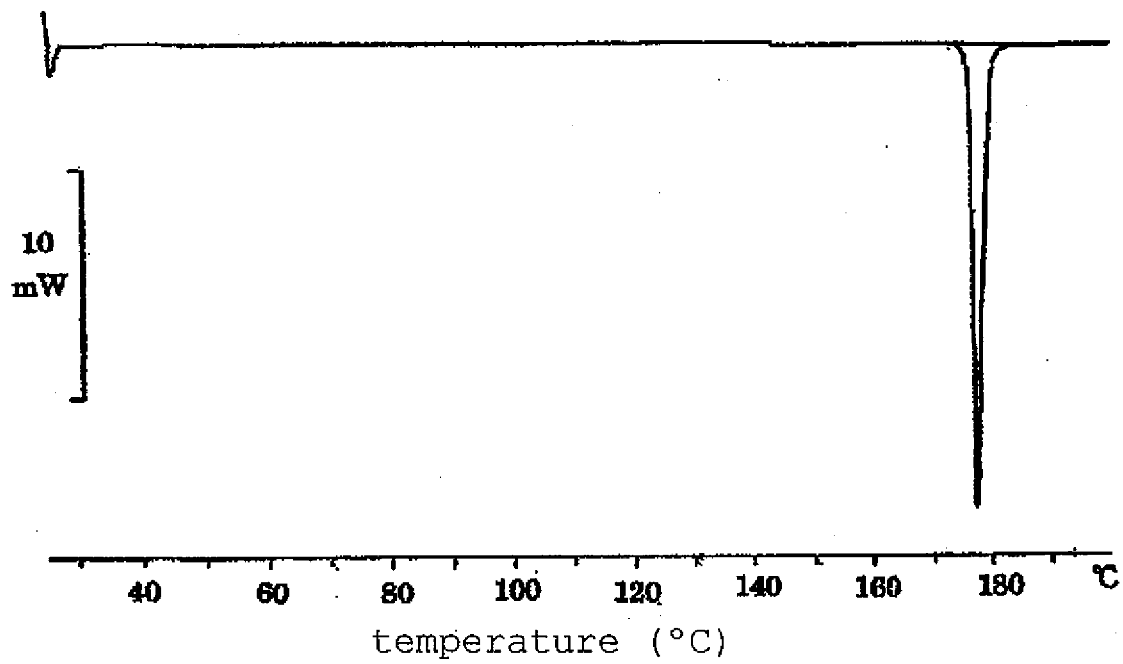

FIG. 3

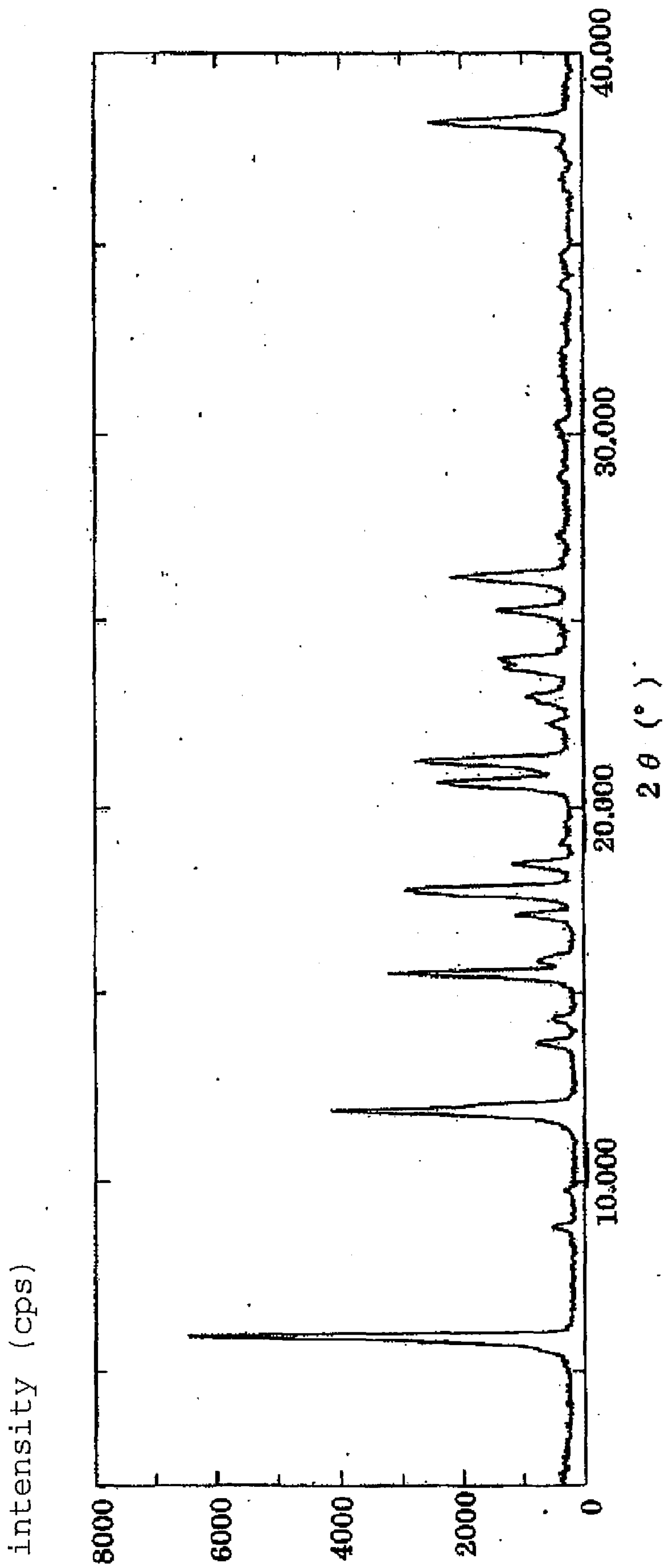
intensity (cps)
8000
6000
4000
2000
0
10.000
20.000
30.000
40.000
2θ (°)

FIG. 4

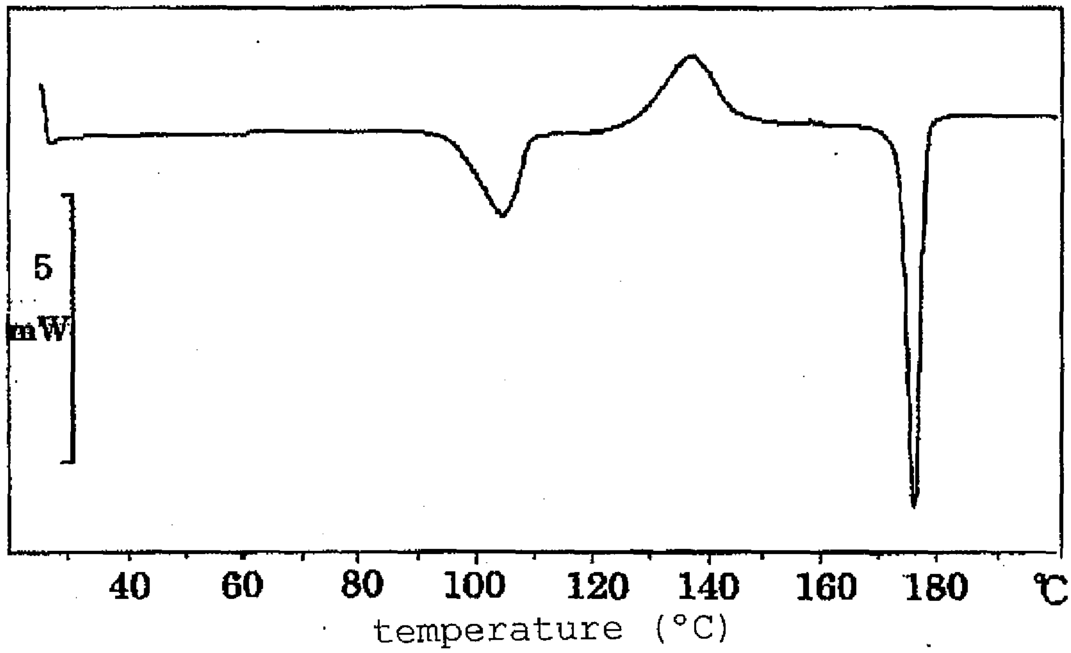

FIG. 5

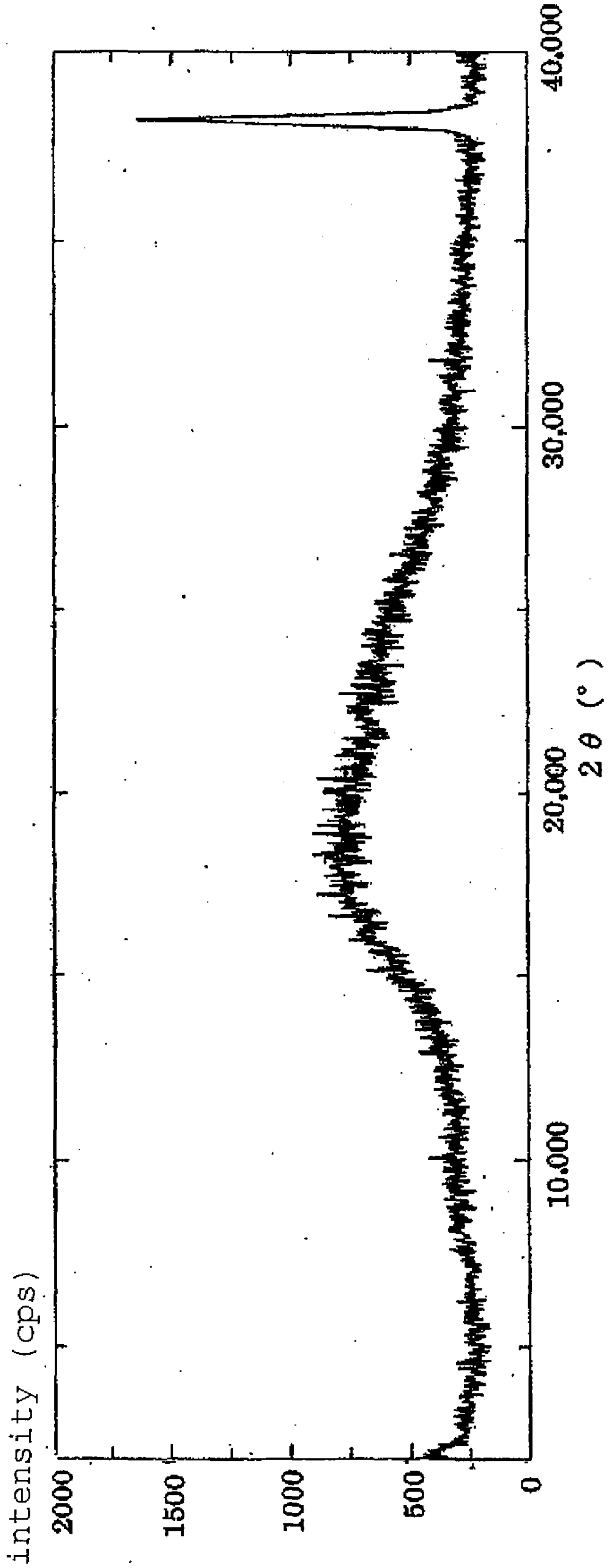
intensity (cps)
2000
1500
1000
500
0
10.000
20.000
30.000
40.000
2θ (°)

FIG. 6

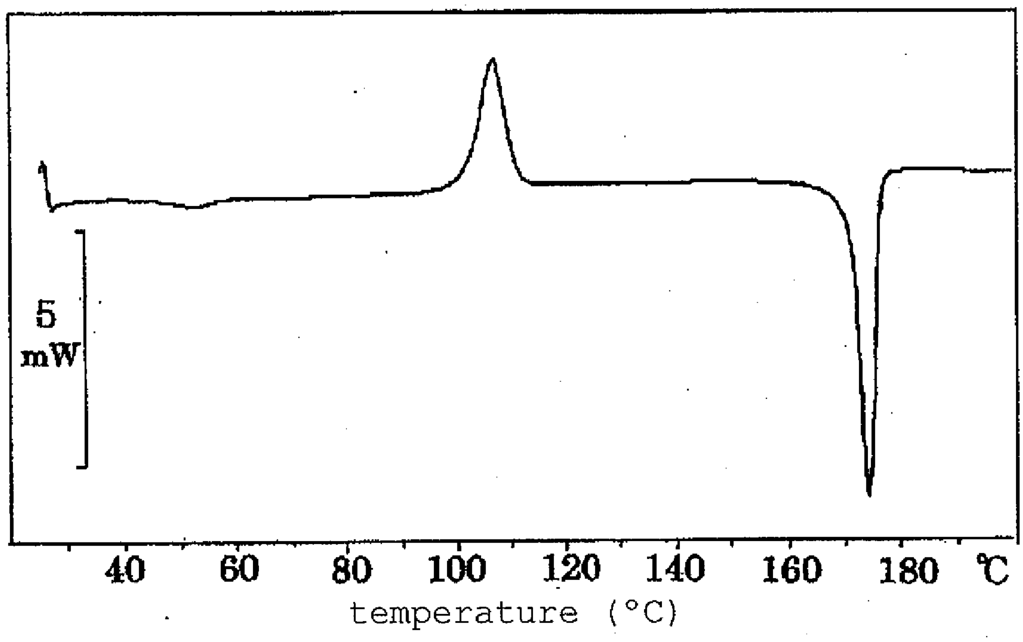
5
mW
40
60
80
100
120
140
160
180
℃
temperature (°C)

FIG. 7

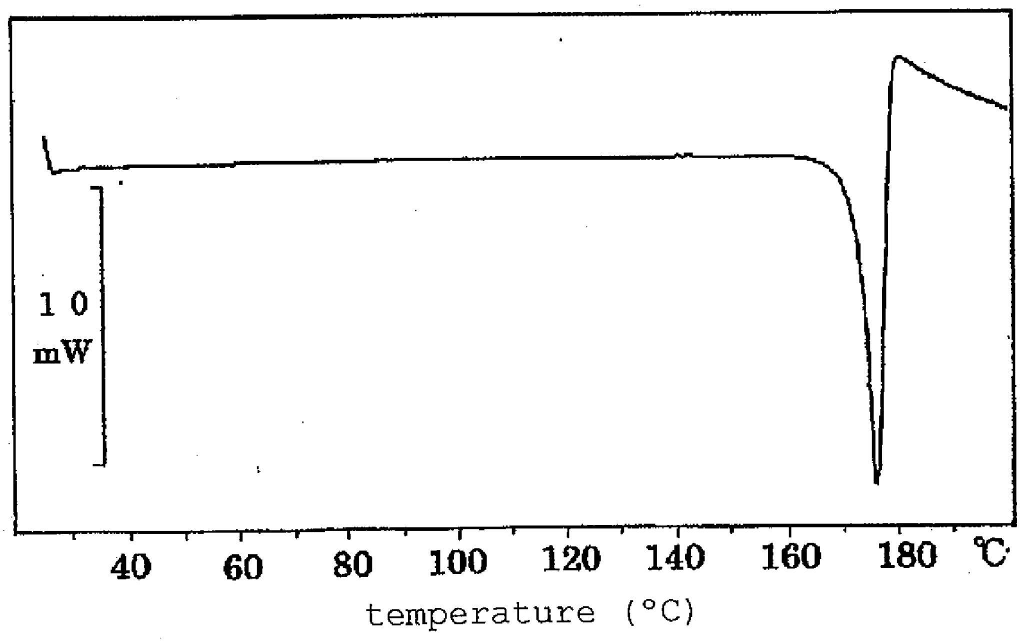
1 0
mW
40
60
80
100
120
140
160
180
℃
temperature (°C)

FIG. 8

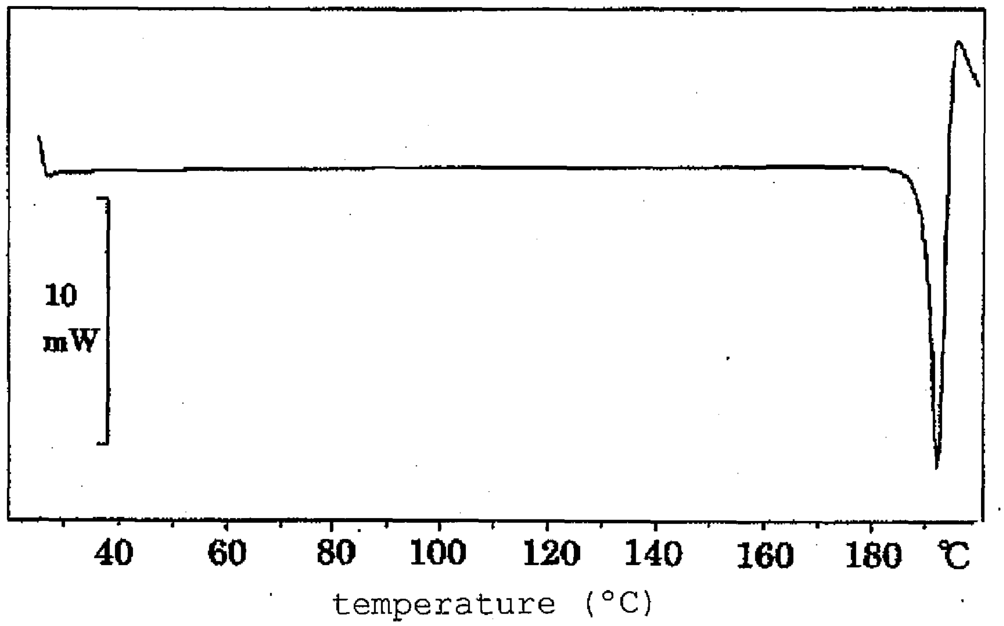
10
mW
40
60
80
100
120
140
160
180
℃
temperature (°C)

FIG. 9

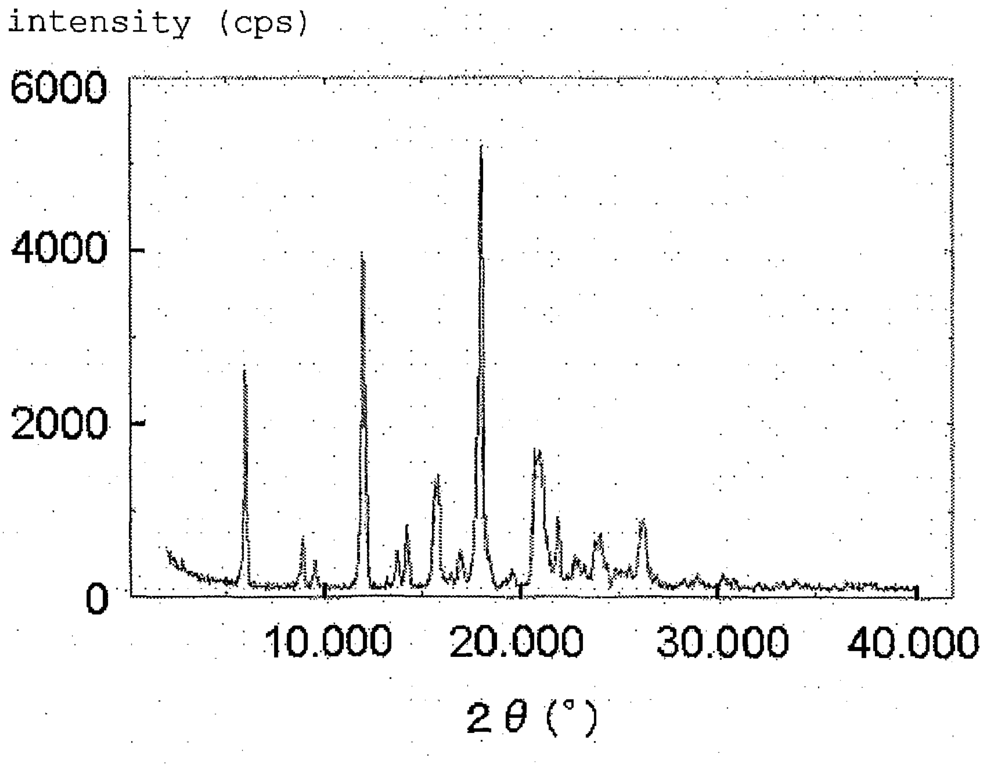
intensity (cps)
6000
4000
2000
0
10.000
20.000
30.000
40.000
2θ(°)

FIG. 10

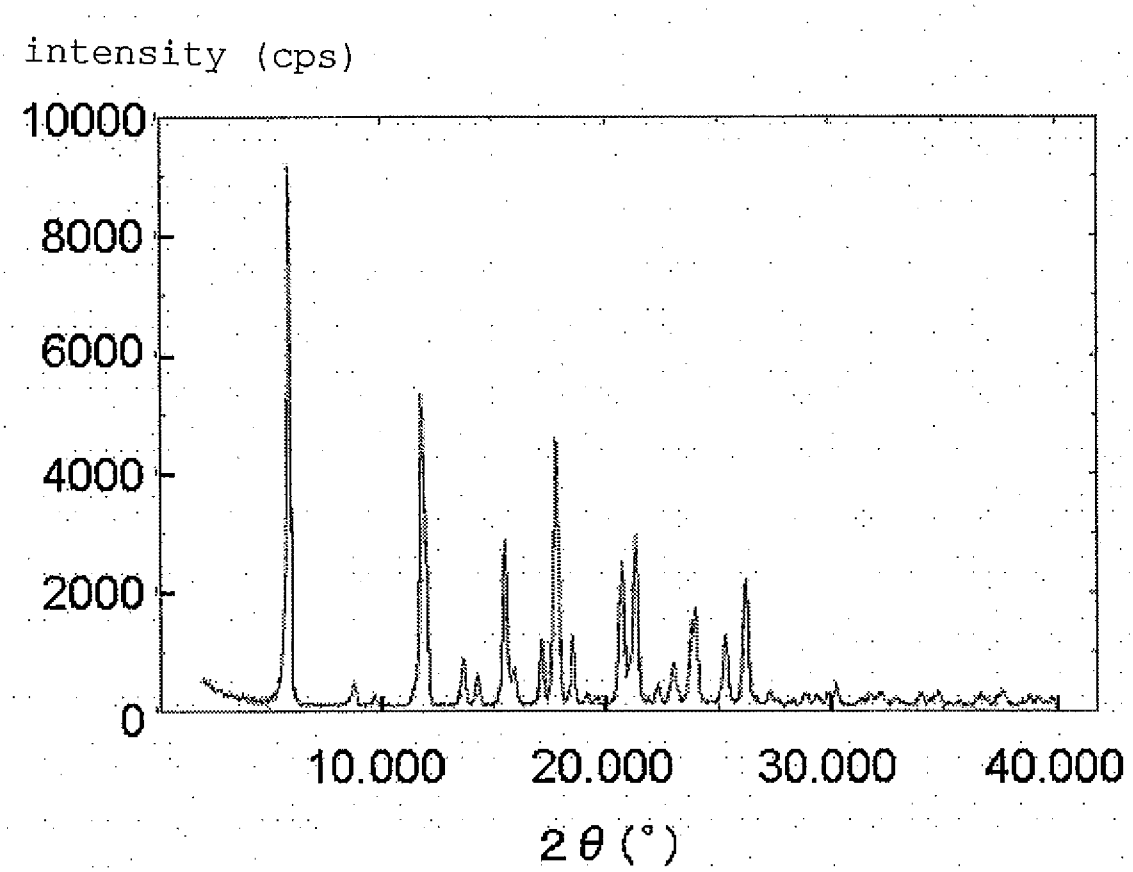
intensity (cps)
10000
8000
6000
4000
2000
0
10.000
20.000
30.000
40.000
2θ(°)

FIG. 11

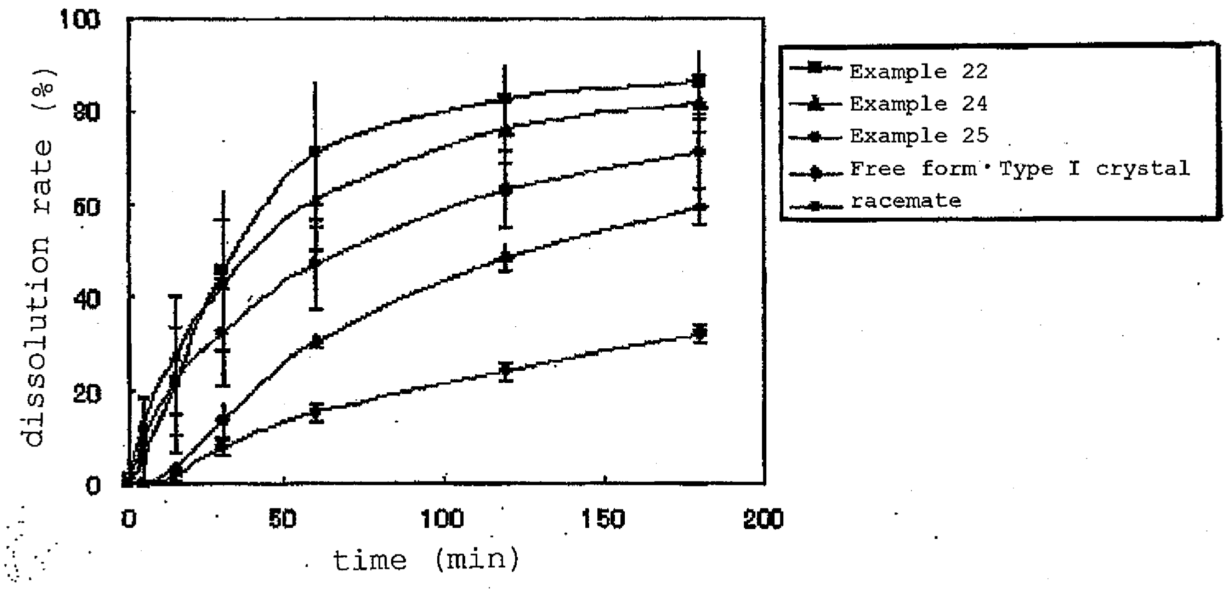
100
80
60
40
20
0
dissolution rate (%)
0
50
100
150
200
time (min)
Example 22
Example 24
Example 25
Free form • Type I crystal
racemate

**INTERNATIONAL SEARCH REPORT**

International application No.
PCT/JP2006/302024

A. CLASSIFICATION OF SUBJECT MATTER
***C07D401/12***(2006.01), ***A61K31/4439***(2006.01), ***A61P1/16***(2006.01), ***A61P1/18***(2006.01), ***A61P9/10***(2006.01), ***A61P11/00***(2006.01), ***A61P13/12***(2006.01), ***A61P17/06***(2006.01), ***A61P19/02***(2006.01), ***A61P25/28***(2006.01),
According to International Patent Classification (IPC) or to both national classification and IPC

B. FIELDS SEARCHED
Minimum documentation searched (classification system followed by classification symbols)
A61K31/4439, A61P1/16, A61P1/18, A61P9/10, A61P11/00, A61P13/12, A61P17/06, A61P19/02, A61P25/28, A61P29/00, A61P31/04, A61P37/06, A61P37/08, C07D401/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | EP 1318140 A1 (Mitsubishi Pharma Corp.), 11 June, 2003 (11.06.03), Abstract; Claims; Par. Nos. [0112], [0113]; example 89; Par. Nos. [0058], [0063], [0078]<br>& WO 2002/22556 A1 & AU 200188045 A<br>& KR 2003059139 A & JP 2002-526757 A<br>& CN 1474803 A & US 2004/138223 A1 | 1-37,39,43, 44<br>38,40-42 |
| X | WO 2000/66566 A1 (SMITHKLINE BEECHAM PLC), 09 November, 2000 (09.11.00), Page 36, line 9<br>& AU 200045567 A & EP 1173424 A1<br>& JP 2002-543189 A | 40 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
15 March, 2006 (15.03.06)

Date of mailing of the international search report
20 March, 2006 (20.03.06)

Name and mailing address of the ISA/
Japanese Patent Office

Authorized officer

Facsimile No.

Telephone No.

Form PCT/ISA/210 (second sheet) (April 2005)

**INTERNATIONAL SEARCH REPORT**

International application No.
PCT/JP2006/302024

C (Continuation). DOCUMENTS CONSIDERED TO BE RELEVANT

| Category* | Citation of document, with indication, where appropriate, of the relevant passages | Relevant to claim No. |
|---|---|---|
| X | EP 1186604 A1 (Takeda Chemical Industries, Ltd.), 13 March, 2002 (13.03.02), Reference Example 227 & WO 2000/76993 A1 & JP 2001-058992 A & AU 200054265 A & JP 2001-503851 A & US 6936602 B1 | 40 |
| X | OVCHARENKO, V.I. et al., Nonclassical Spin Transitions, Journal of Structural Chemistry, 2002, Vol.43, No.1, pages 153 to 167, 155 | 40 |
| A | JP 05-331167 A (Director General of National Institute of Health Science), 14 December, 1993 (14.12.93) & JP 94051699 B2 | 1-44 |
| P,A | JP 2005-120027 A (Mitsubishi Pharma Corp.), 12 May, 2005 (12.05.05) (Family: none) | 1-44 |

Form PCT/ISA/210 (continuation of second sheet) (April 2005)

INTERNATIONAL SEARCH REPORT

International application No.
PCT/JP2006/302024

Continuation of A. CLASSIFICATION OF SUBJECT MATTER
(International Patent Classification (IPC))

***A61P29/00***(2006.01), ***A61P31/04***(2006.01), ***A61P37/06***(2006.01), ***A61P37/08***(2006.01), ***C07D231/12***(2006.01), ***C07D401/14***(2006.01)

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0222556 A
- JP 2005029907 A


### Non-patent literature cited in the description


- *Annu. Rev. Immunol.,* 1994, vol. 12, 775-808
- *Immunopharmacology,* 1997, vol. 38, 3-15
- *Curr. Pharm. Des.,* 1999, vol. 5, 737-755
- *IDrugs,* 1999, vol. 2, 686-693
- *Curr.Pharm.Des.,* 1999, vol. 5, 737-755
- *J. AM. CHEM. SOC.,* 2002, vol. 124, 7421-7428